(19) Europäisches Patentamt
European Patent Office
Office européen des brevets

(11) EP 3 695 782 A2

(12) EUROPEAN PATENT APPLICATION

(43) Date of publication:
19.08.2020 Bulletin 2020/34

(51) Int Cl.:
A61B 5/087 (2006.01)     A61B 5/00 (2006.01)
A61B 7/00 (2006.01)      A61B 5/0205 (2006.01)

(21) Application number: 20164320.2

(22) Date of filing: 04.10.2005

(84) Designated Contracting States:
AT BE BG CH CY CZ DE DK EE ES FI FR GB GR
HU IE IS IT LI LT LU LV MC NL PL PT RO SE SI
SK TR

(30) Priority: 04.10.2004 US 615813 P

(62) Document number(s) of the earlier application(s) in
accordance with Art. 76 EPC:
05789090.7 / 1 861 014

(71) Applicant: SpiroFriends Technology ApS
2800 Kgs. Lyngby (DK)

(72) Inventor: AVRAHAMSON, Dan
2800 Kongens Lyngby (DK)

(74) Representative: D Young & Co LLP
120 Holborn
London EC1N 2DY (GB)

Remarks:
This application was filed on 19.03.2020 as a
divisional application to the application mentioned
under INID code 62.

(54) HANDHELD HOME MONITORING SENSORS NETWORK DEVICE AND METHOD

(57)    Light weight personal handheld home monitoring and managing device, which includes a Sound Sensor network/array of Sound Sensor networks combined with an Artificial Neural Network (ANN) and a build in system and methods, making this device an intelligent and portable apparatus to address specific health issues.

The combined apparatus is used for managing and/or guidance and/or diagnosing and/or controlling and managing purposes. This present version of the apparatus will address pulmonary disorders and diseases or similar ailments.

Figure 1A

EP 3 695 782 A2

**Description**

Description is made for the purpose of illustrating the general principles of the invention and should not be taken in a limiting sense.

**FIELD**

The invention relates to a light weight personal handheld home monitoring and managing device, which includes a Sound Sensor network/array of Sound Sensor networks combined with an Artificial Neural Network (ANN) and a build in system and methods, making this device an intelligent and portable apparatus to address specific health issues. The combined apparatus is used for imaging and/or guidance and/or diagnosing and/or controlling and managing purposes. This present version of the apparatus will address pulmonary disorders and diseases or similar ailments.

**BACKGROUND**

Asthma is one of the most common pulmonary chronic diseases in the world, found in both adults and in children. In all parts of the world asthma is a fast growing disease. The prevalence of asthma increases as communities adopt western lifestyles and become urbanized.

According to the latest report from the Global Initiative for Asthma (GINA) it is estimated that as many as 300 million people of all ages and all ethnic backgrounds suffer from asthma, and that the burden of this disease to governments, health care systems, families, and users is increasing worldwide. It is also estimated, that there may be an additional 100 million persons with asthma by year 2025.

Asthma is considered a chronic pulmonary disease as of today no cure exists, but stabilizing the disease by the right medication at the right time will preserve the life quality for human beings.

Accordingly pre- and post- diagnosis, home monitoring of the pulmonary function is instituted by the general practitioner. This will include repeatedly daily measurements of the pulmonary function and timely logging of data and symptoms, and is considered very essential for the general practitioner to make a precise diagnosis, choose the right treatment, and decide on the medication.

Diagnosed asthmatic users are very dependent upon an acceptable control of their disease and the management of the day-to-day adjustment of their medication.

The well known problems with asthmatic home monitoring today are the users lack of compliance, the wrong use and the lack of precision in the devices used for the home measurements, all in all causing reduced life quality for the user when asthma is not stabilized.

This invention will reduce errors in measurements as the device uses a network of Sound Sensors and a self correlating system in an Artificial Neural Network to analyze on the collected physiological data originating from a specific users diseases behavior pattern and its pattern recognition.
The device compares these data with data previously collected and stored by the device about the same user and/or loaded calibrated information stored about the user and/or loaded reference information based upon information from background population. This will make a unique accurate picture of the users true disease based on behavior pattern and the will learn and predict attacks such as asthma attacks.

The recorded data from user diseases behavior pattern will be stored in the devices storage unit continuously with a set of date and time registration details. To shorten learning time for artificial neural network unit, there is possible to upload user's data into the device. Similarly earlier or pre-measured data can be uploaded to the device.

The learning of user diseases behavior pattern and increase the level of compliance, by objective logging of the user condition in an easy manner and making suggestions to support the self-control of medicine to take.

This invention also deals with a cooperative calculation approach for using artificial neural network ensembles and applies multi objective optimization.

Cooperative calculation approach is a recent paradigm in evolutionary computation that allows through learning how to modeling the lungs sound and the acoustic of respiratory passage and its cooperative environments for a specific user in relation to itself. Although processing algorithms that make the device able to handle with its artificial neural network and with a sufficient number of neurons in the hidden layer would suffice to solve user diseases behavior pattern and indicate or alarm, appearance of a given condition for the user for example an asthmatic attack.

**SUMMARY**

The invention relates to a light weight personal handheld home monitoring device, with an in build network of Sound Sensors, artificial neural network system, intelligence and portable device, system and method addressing a specific health issue.

The system and method is used for imaging and/or guidance and/or diagnosing and/or controlling purposes. The main aspect of present version will address pulmonary normally health care, disorder and diseases or similar healthy or ailments conditions.

The invention relates to a method and system for specific recognition of lungs function conditions, especially the presence of specific sound(s) that can be related to a given specific lungs function condition with user himself as reference. That means an easy accurate method to learn and recognizing a given user disease behavior pattern and accuracy condition to compare with user itself.

For this purpose, a special network of sound sensors is introduced into the breathing apparatus and by preprogrammed sounds providing information about sound signatures, which will be stored for analysis. When certain conditions are detected, actions such as activating an alarm signal are carried out. A simultaneous control of the signal by a user is made possible by rep resenting the signals on a display or by the output thereof on loud speaker/headphones or the like.

The device, system and method are specially designed for improvements in the early diagnosis and prevention of Pulmonary Function Disorder and Diseases (PFDD), in particular Asthma and COPD.

The device is generally capable to be used as an electronically journal, data acquisition, storage of measurements, medicine optimizer, storage of keyed input data and user health and diseases pattern behavior.
Using the device in the ongoing measurement, the devise will after a period analyze the measurements stored in form of data, and set some indications FLAGS that the general practitioner can use in his overall diagnosis, treatment and choice of medication.

The device is able to establish communication to a data processor through a wire or wireless connection (for example USB, Bluetooth, Infrared etc.) and transmit and receive data/information to and from the data processor via a software Interface. The measured, data or information can also be shown as real time curves and plots.

As the device is flexible in its design, it will be independent upon the type of disease to acquire data/information about. This means that the equipment can be adapted for several types of measurements, just by changing the loaded software with respect to the used Sound Sensor Networks.

For example a system and method for testing and recording the peak expiratory flow rate (PEFR), forced expiratory volume ($FEV_1$), ($FEV_6$) and forced volume capacity (FVC) of a asthmatic user, comprises (I) prompting the user to cause the sensing of the expiratory and/or inhalation flow rate, (II) sensing the expiratory and/or inhalation flow rate of the user and (III) generating a signal representative of this biological condition, (IV) processing the signal tb generate biological data representative of the biological condition, (V) generating date and time stamp data representative to the date and time when the biological condition was sensed, (VI) storing the biological data and the date and time data, and (VII) retrieving the stored biological data together with the time stamp data (VIII) analyze the stored information using an artificial intelligence analytic method, (IX) display the result of the analysis, (X) store the result for later reference and analysis.

Methods for using the Sound Sensor Network device, system and methods for making the sensor device also are disclosed.


**BRIEF DESCRIPTION OF THE DRAWINGS**

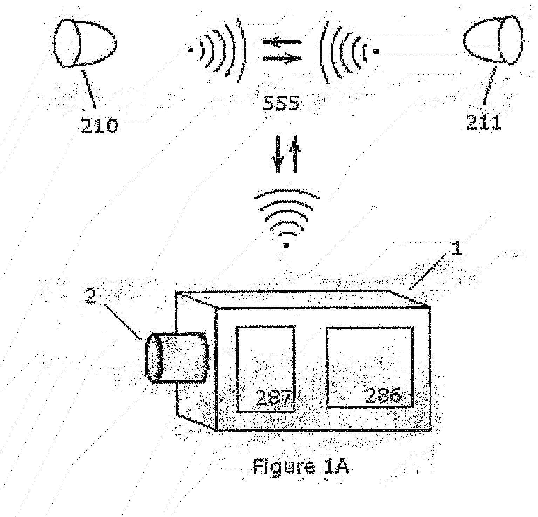

Fig. 1A        the main device with it two external ear devices

The following numbers refer to:

Fig. 1A-1        an example of main devices physical dimension
Fig. 1A-210      the right ear device
Fig. 1A-211      the left ear device
Fig. 1A-555      wireless bidirectional interconnection between left and/or right ear devices and/or main device

Fig. 1B        Illustrates the main devices internal block diagram

The following numbers refer to:

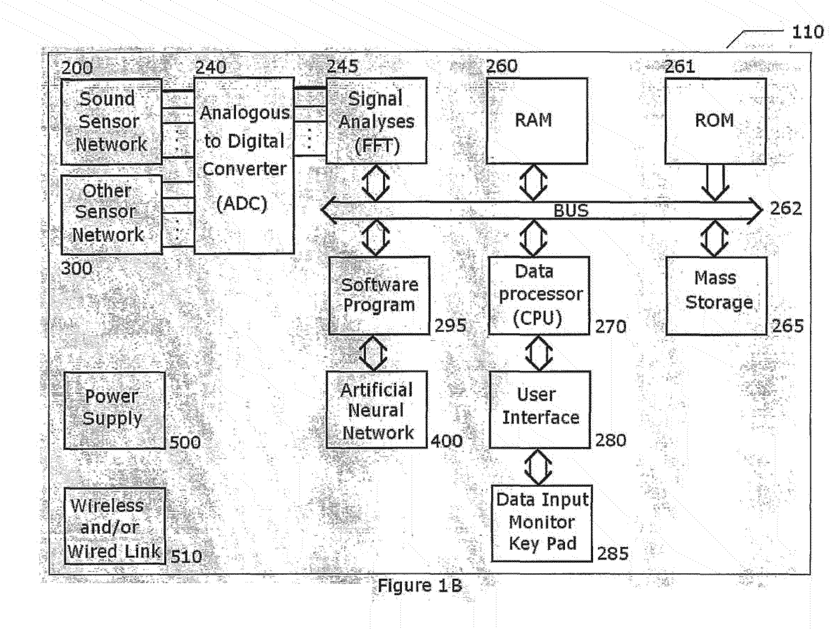

Fig.1B-1        The main devices block diagram overview

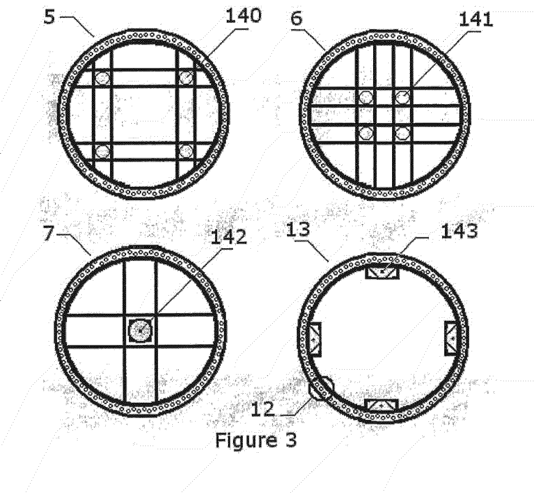

Fig.1B-200.      The sound sensor network as shown in Fig. 3, 4 and 8.
Fig.1B-240.      The analog to digital converter
Fig.1B-245.      The signal analysis process connected to the data bus

Fig.1B-260.   The random accessible memory connected to the data bus

Fig.1B-261.   The read only memory storing connected to the data bus

Fig.1B-262.   The data bus connecting the various blocks

Fig.1B-265.   The mass storage for the captured sensor data

Fig.1B-270.   The data processor

Fig.1B-280.   The user interface

Fig.1B-285.   The data input monitor and key pad.

Fig.1B-295.   The software program that is developed for the device.

Fig.1B-300.   Other sensors connected to ADC

Fig.1B-400.   The artificial neural network

Fig.1B-500.   the power supply of the devise

Fig.1B-510.   The external interface wireless and/or wired link.

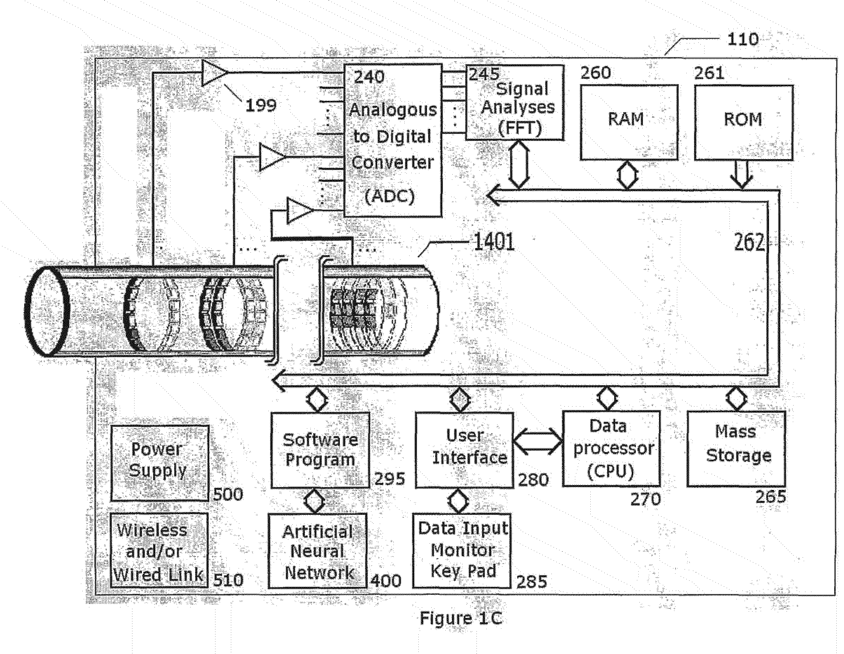

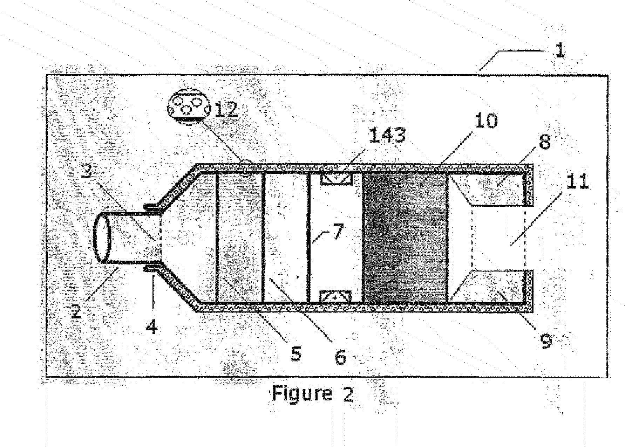

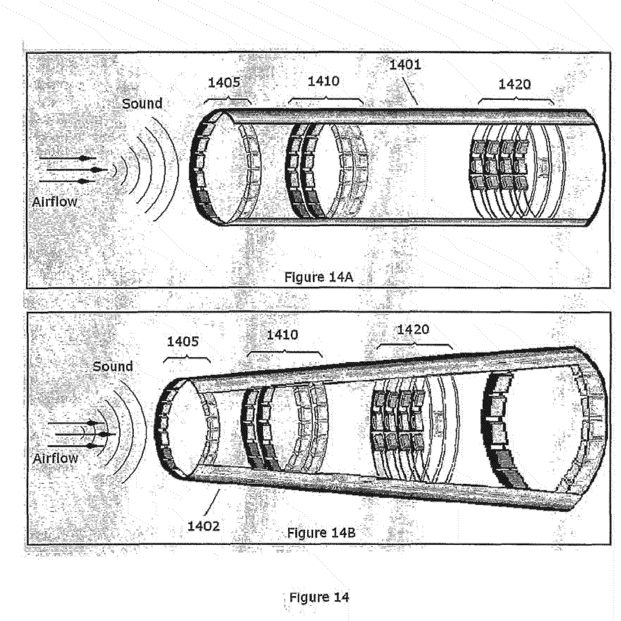

Fig. 1C    Illustrates of fig.1A (1), its contain fig.1B (110), tube with sensor network fig.2 and fig.14, mouthpiece and filter

The following drawing numbers refer to:

Fig. 1C-199   Illustrate an amplifier, which is matched to it's respectively sensor

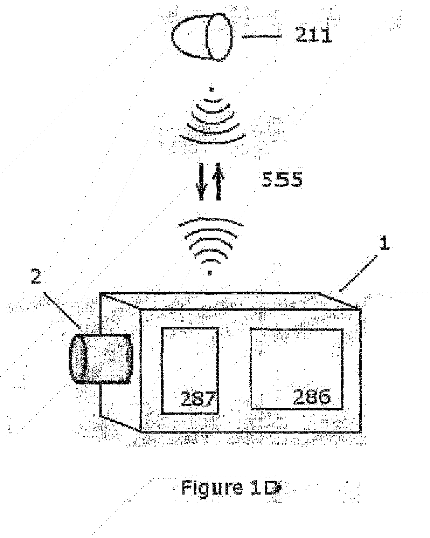

Fig. 1D      the main device with one ear devices option (Left or Right)

FIG. 2       Illustrates an example of a detailed cross sectional view of the front end and the back end of the Sound Sensor network device.

The following drawing numbers refer to:

Fig. 2-2.        Exchangeable mouthpiece

Fig. 2-3.        Exchangeable micro filter to capture moisture, dust, bacteria and similar particles

Fig. 2-4.        Socket to keep Mouthpiece and its filter and changeable mouthpiece

Fig. 2-5.        Sound Sensor Network (SSN)

Fig. 2-5+6+7.   Array of Sound Sensor Networks (ASSN)

Fig. 2-8+9.     Hardware and software such as Artificial Intelligence (AI), memory, data processor like device, add on memories, other electronics accessories and rechargeable battery

Fig. 2-10.      Changeable medico technique module

Fig. 2-11.      Bidirectional Air Flow Detector (BAFD), Flow Meter (FM), breathing frequent counter and output ventilation port

Fig. 2-12.      Sound damping material, to minimize common mode sound and noise

Fig. 2-143.     Sound sensors for elimination of common mode sound and noise

FIG. 3     illustrates cross-sectional views of the sound sensor network build into the airflow tube. This view is referenced to the cross-sectional view as illustrated in FIG. 2.

The following drawing numbers refer to:

Fig. 3-5.     The first level of Sound Sensors

Fig. 3-6.     The second level of Sound Sensors

Fig. 3-7.     The third level of Sound Sensors.

Fig. 3-12.    The sound damping material, to minimize common mode sound and noise

Fig. 3-13.    The block of Sound Sensors to eliminate ambient and common mode sound and noise

Fig. 3-140    The 4 sound sensors placed in the first network layer

Fig. 3-141    The 4 sound sensors placed in the second network layer

Fig. 3-142    The center sound sensor third layer.

Fig. 3-143    The 4 sound sensors to eliminate ambient and common mode sound and noise.

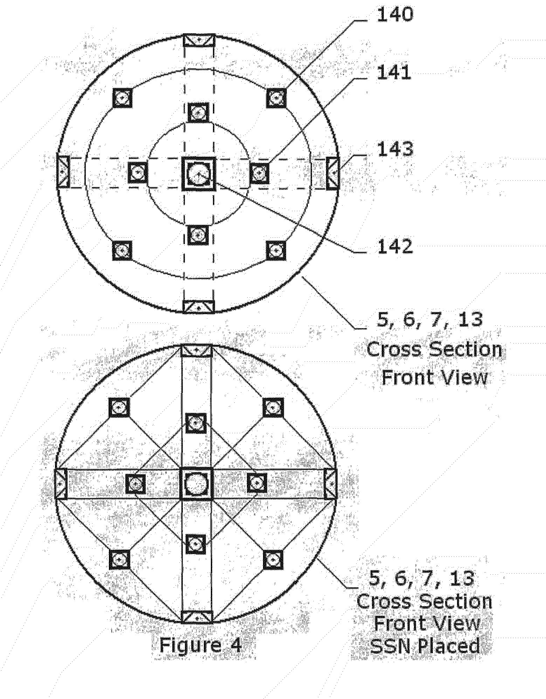

FIG. 4     illustrates a perspective front view of the sound sensor network as seen from the front end of the airflow tube as illustrated in FIG. 2.

The following drawing numbers refer to:

Fig. 4-140    the 4 sound/other sensors placed in the first network layer

Fig. 4-141    the 4 sound/other sensors placed in the second network layer

Fig. 4-142    the aerodynamic sound sensor third layer at center

Fig. 4-143    The 4 sound sensors to eliminate ambient and common'mode Sound and noise.

Fig. 4-5    the first level of Sound Sensors

Fig. 4-6    the second level of Sound Sensor

Fig. 4-7    the third level of Sound Sensors.

Fig. 4-13    the block of Sound Sensors to eliminate ambient and common mode sound and noise

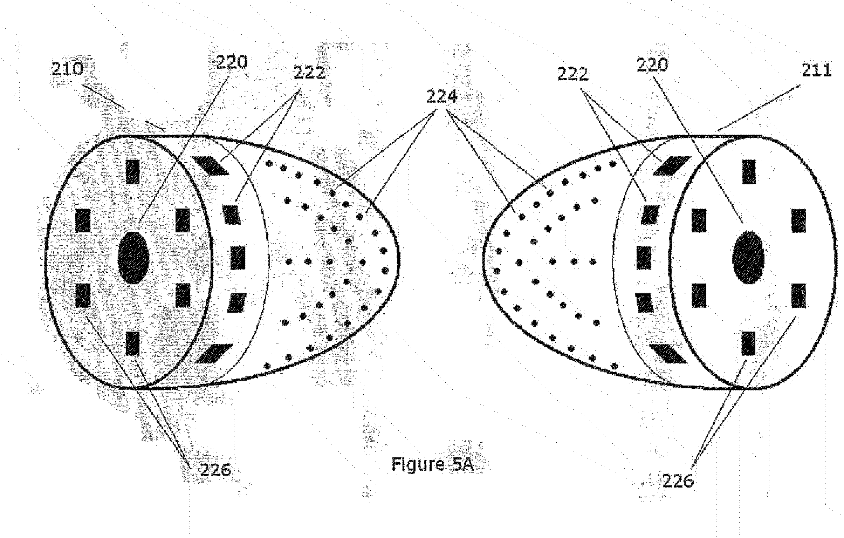

FIG. 5A    The ear devices, the right ear device 210 and the left ear device

The following drawing numbers refer to:

FIG.5A-220    Sound and/or Temperature and/or Pressure sensors

FIG.5A-222    Sound and/or Temperature and/or Pressure sensors

FIG.5A-224    Sound reproducer (not shown) and the Sensor network consist of: Temperature and/or Pressure and/ or Moisture and/or Sound Sensors

FIG.5A-226    Sound and/or Temperature and/or Pressure sensors

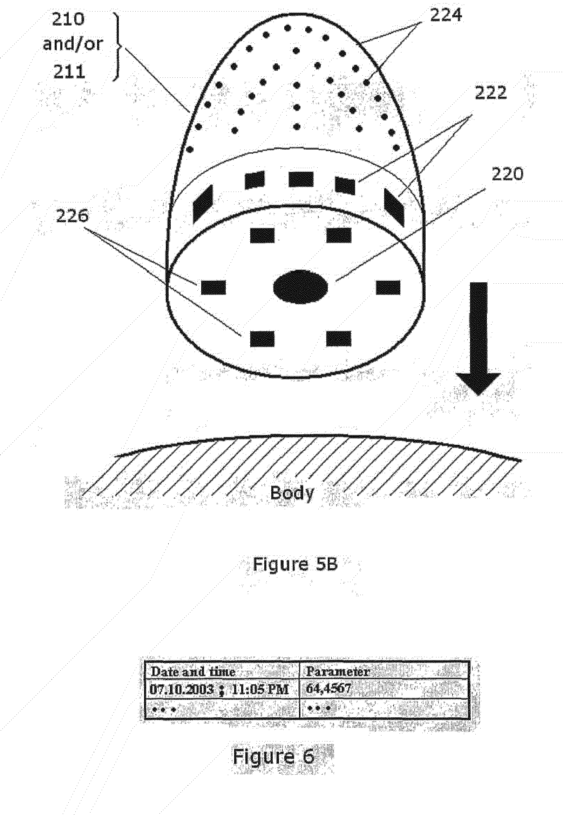

FIG. 5B    How to place the ear device or devices on the body

The following drawing numbers refer to:
As mentioned in figure 5A

FIG. 6    Illustrates an example of a data set

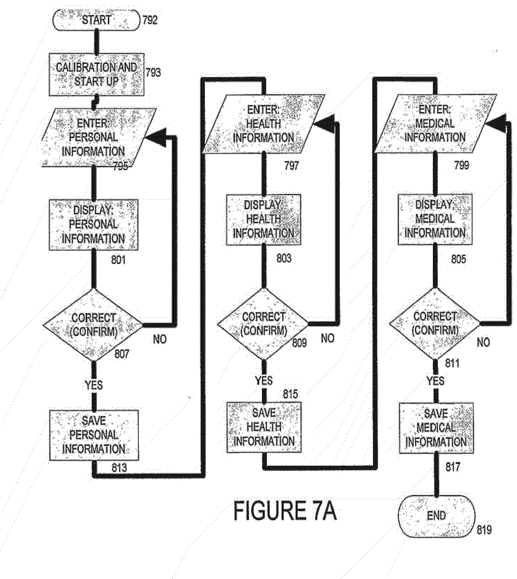

FIGURE 7A    Illustrates subroutine for initial data input

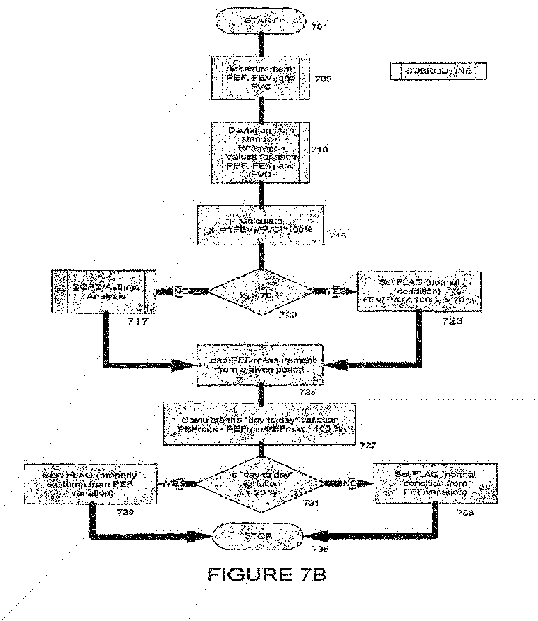

FIGURE 7B    Illustrates subroutine for COPD/asthma diagnosing.

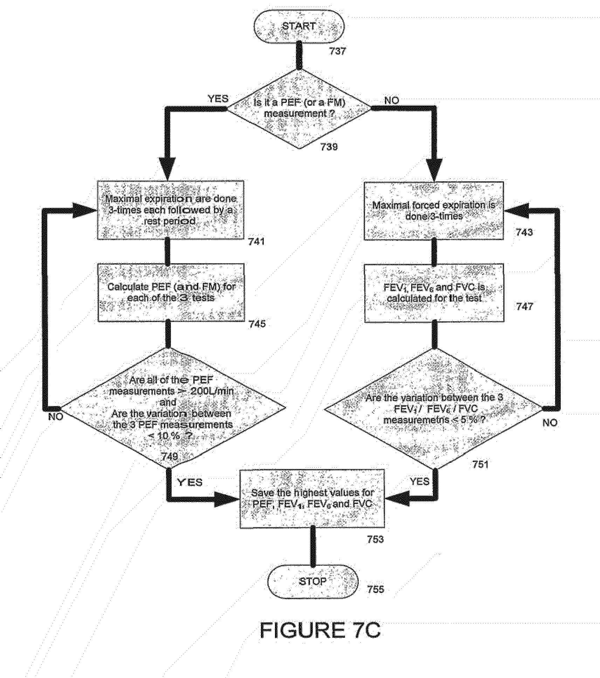

FIGURE 7C    Illustrates subroutine for measurement of PEF, $FEV_1$, $FEV_6$ and FVC.

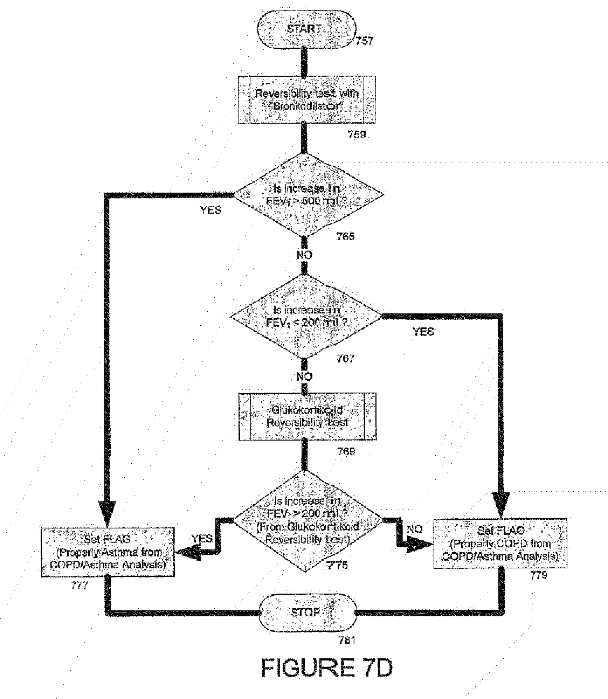

FIGURE 7D    Illustrates subroutine for Reversibility with emphasis in COPD/asthma diagnosing

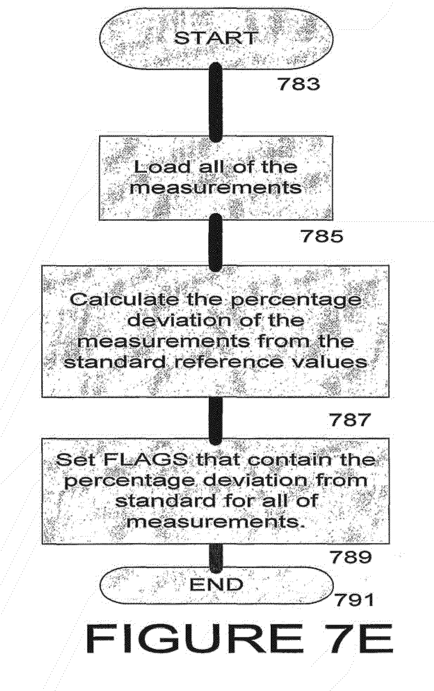

FIGURE 7E    Illustrates subroutine for user data/information managing and calculating the percentage deviation of the measurements from the standard reference values and Sound Sensor network calibration via ANN.

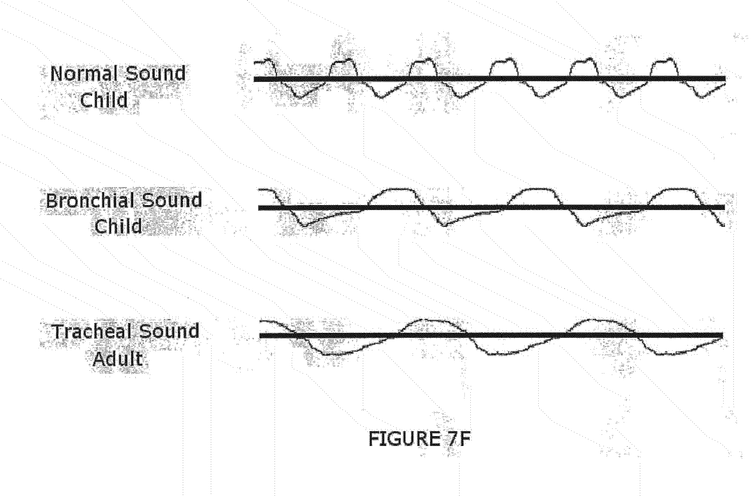

FIG. 7.F    Illustrates lung sound examples

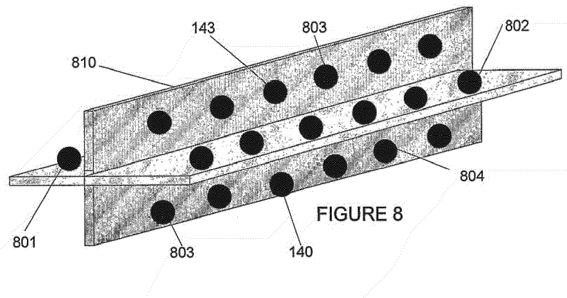

FIG. 8    Illustrates the alternative of Sound Sensor Network an other sensor network implementation (as in figure 5, and the Sensor network consist of: Temperature and/or Pressure and/ or Moisture and/or Sound Sensors, and flow meter)

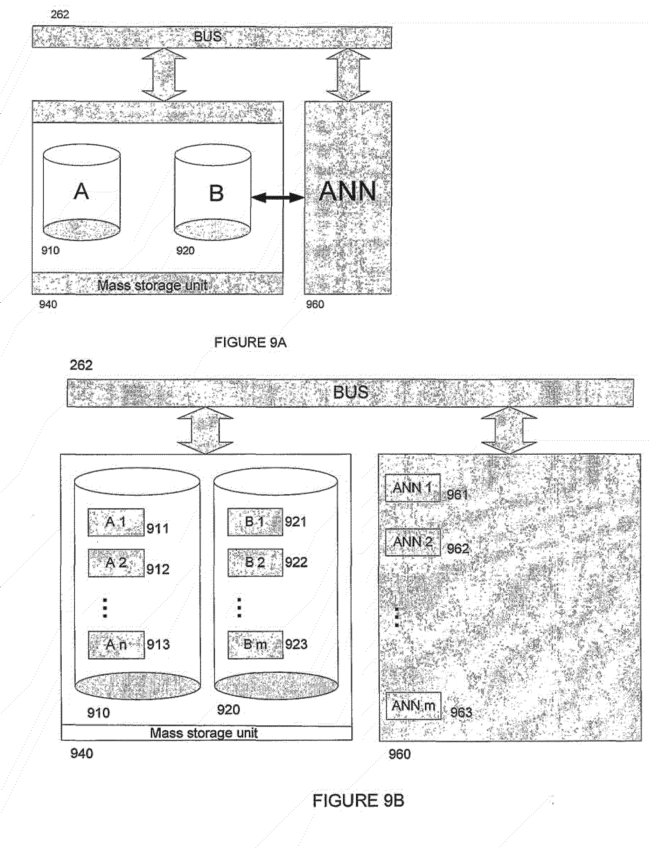

FIG. 9A    illustrates Mass Storage Unit, where data is stored, databus and ANN unit

FIG. 9B    Illustrates Mass Storage Unit, where data is stored, databus and ANN unit with more detailed separation between unit A and B.

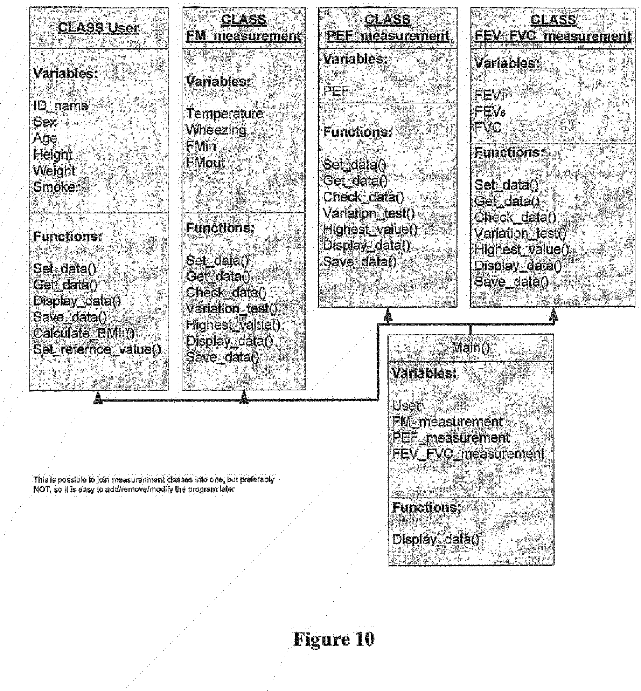

Fig. 10    illustrates a software implementations example

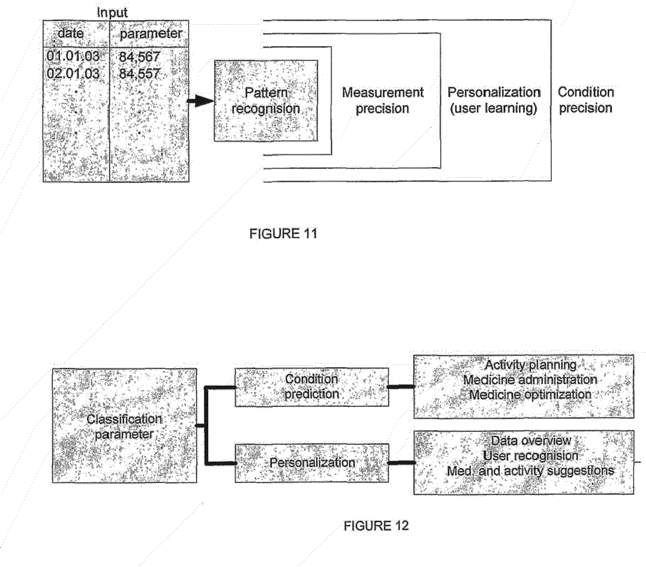

Fig. 11    an example on from data input set to condition prediction and ANN classification

Fig. 12    An example on an activate plan and ANN output data set

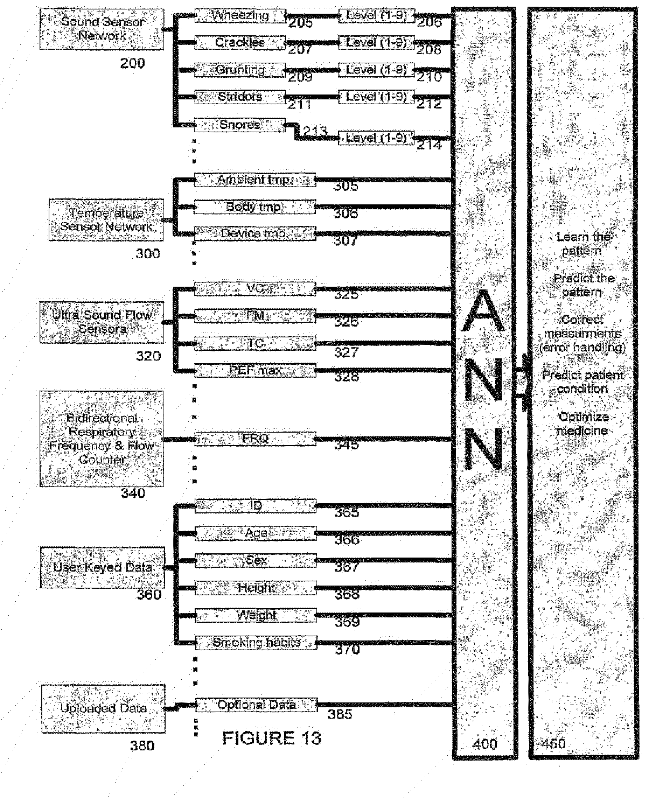

Fig. 13    ANN and the Sound Sensor network groups, G1 to G6 and ANN output data

Fig. 14A    Illustrates of an alternative to Sound Sensor and other sensor network as in fig. 2 and fig. 5A and B (an internal view of another embodiment of the both Sound Sensor network and other Sensor networks).

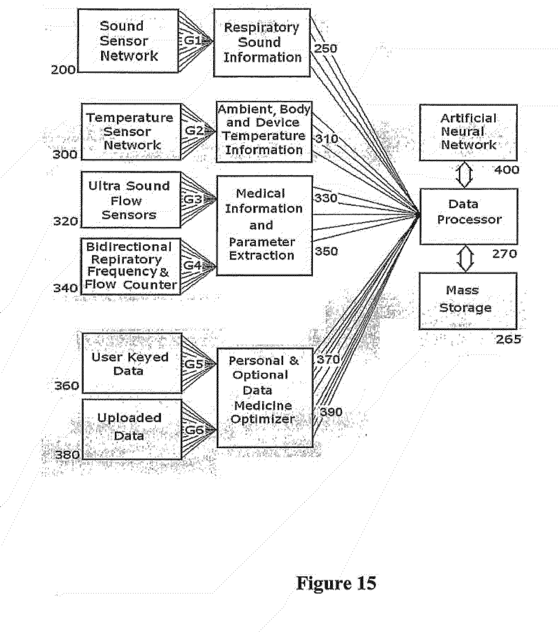

Fig. 15    ANN and the Sound Sensor network groups, G1 to G6 and data processor.

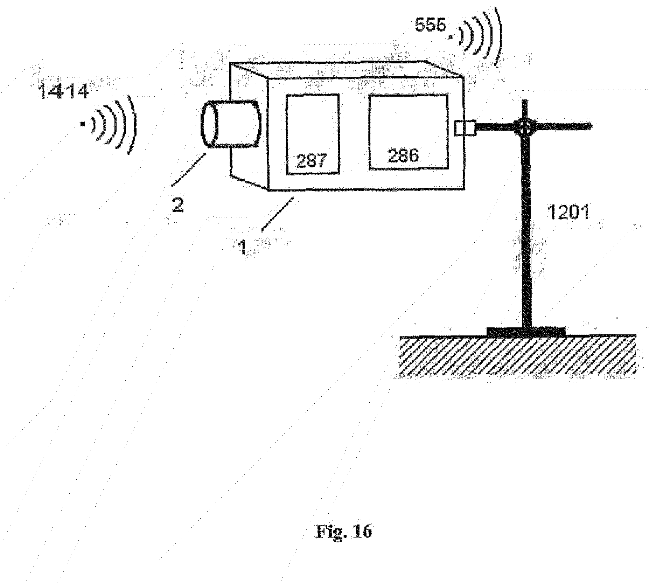

Fig.16    Shows a set up of the mother device (as well as the ear device). Block 1414 shows a sound signal. The apparatus is able to analyze an incoming signal and to transmit a result as an example Supervision of a patient or user. Block 1201 shows a stand for holding the device.

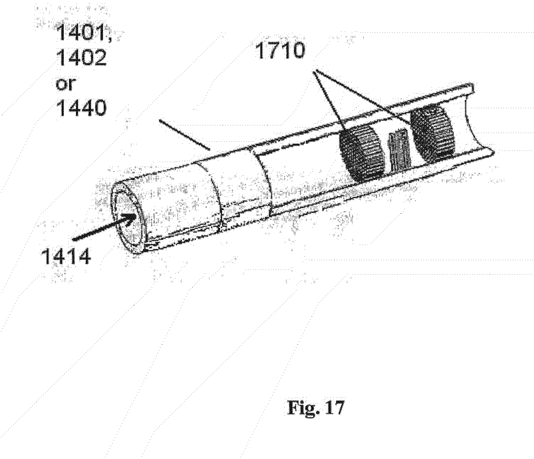

Fig. 17    Illustrates an embodiment with two discs placed inside the mouth piece to achieve a uniform velocity profile. The blocks 1710 show the discs comprising small longitude pipes.

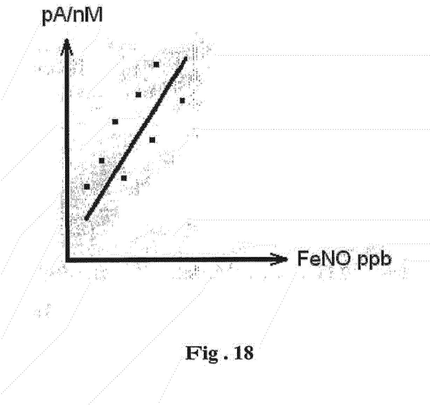

Fig. 18.    Shows the sensor output signal as a function of NO density.

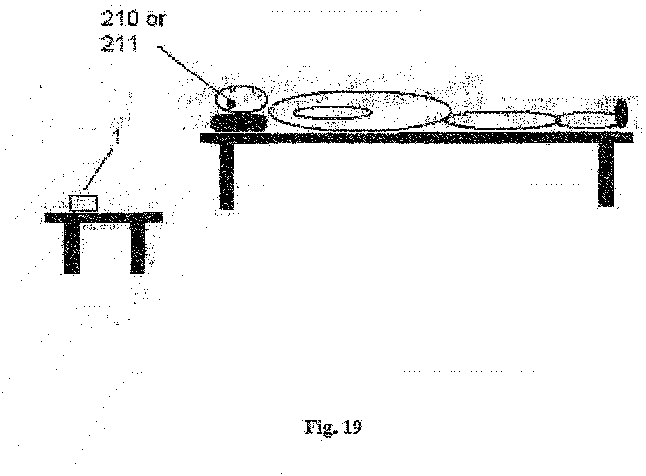

Fig 19    shows a user wearing an ear device or ear devices and the mother device is placed closely to the bed. This principle is used for supervision, snore-detection, apnea-detecting for simply for learning.

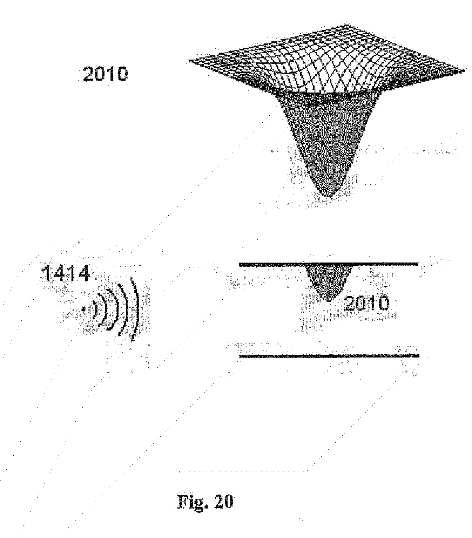

Fig. 20    Illustrates a Gaussian surface, block 2010, and placement of the Gaussian surface in the tube in a cross-section view. Block 1414 shows an exhaled breath moisturized gas.

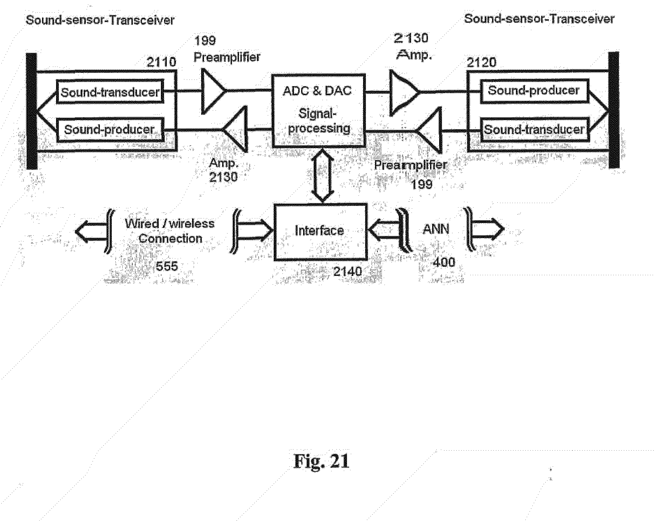

Fig 21    Illustrates a combined sound transducer and sound producer. They act as half-duplex-sound-units. This principle shows an ear device which can function both ways e.g. both for warning the user and for receiving a signal from outside. Block 555 shows that the ear device can also communicate with a mother device, a PC or the like wirelessly.

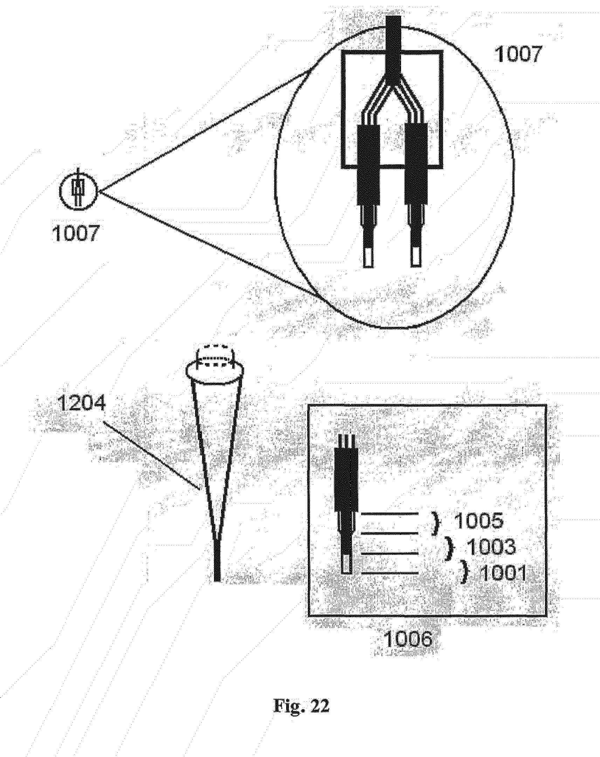

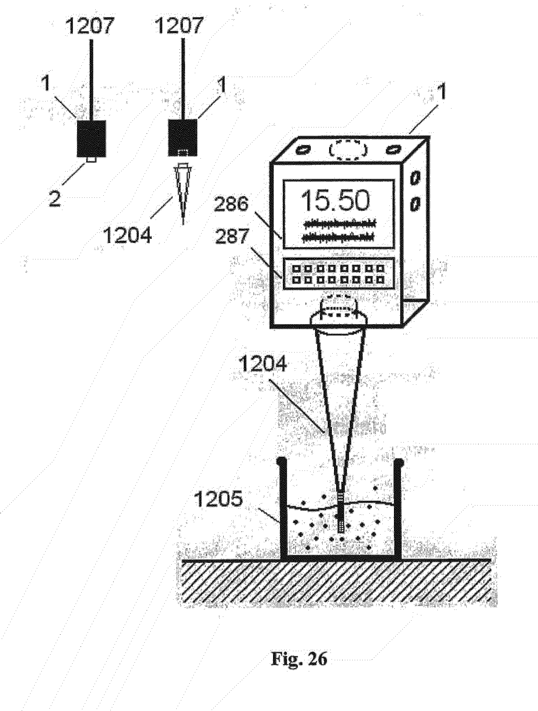

Fig. 22    Shows a biogas sensor (BS) that measures NO. Module 107 shows either a double sensor of the same biogas sensor or a different sensor such as a temperature or biogas sensor. Module 1204 shows the application of such a sensor. Module 1204 can be coupled with the mother device for measuring liquid and condensate (see fig. 26).

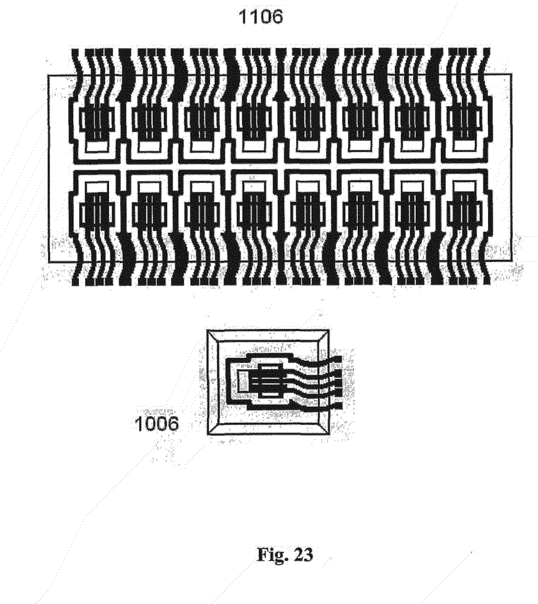

Fig. 23    Shows a micro chip biogas sensor. It shows also a planar structure opposite of the sensor as shown in fig. 22.

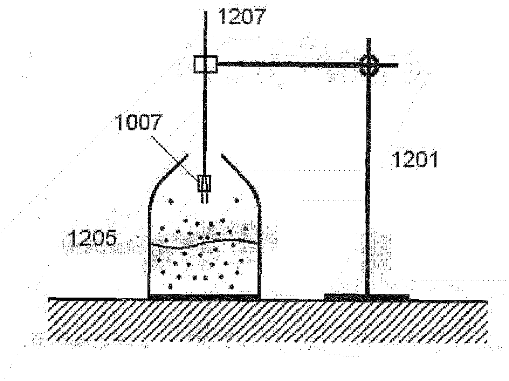

Fig. 24    Illustrates the use of the biogas sensor for measurements of gasses by mixing exhaled condensate with exhaled breath condensate. Another liquid, distilled water, with enzymes and/or enzymatic solutions releases biogases, and after that the sensor can measure the contents of the biogases.

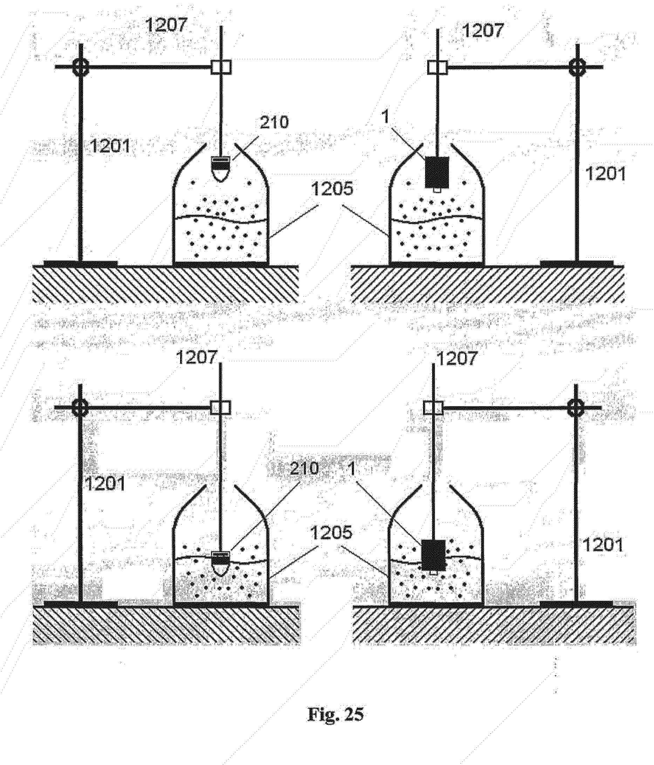

Fig. 25    shows the same principle as in fig. 24, but here is the mother device used as well as the ear device is used for measuring biogases. It also shows that the ear device and the mother device can directly measure liquid components such as pH, dissolved NO and/or other biomarkers.

Fig. 26    Illustrates how it is possible to remove the mouthpiece of the mother device and to connect a liquid micro sensor.

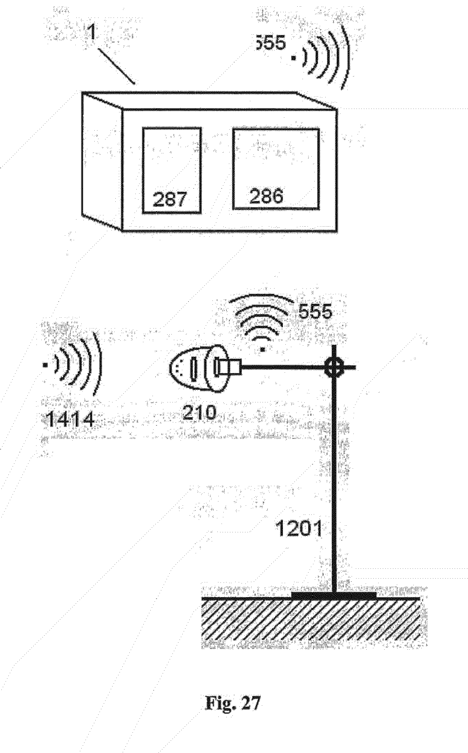

Fig. 27    Illustrates how to fasten an ear device to a stand and to collect the sound information signals form the environment and transmitting them to the mother device or to other wirelessly paired terminal.

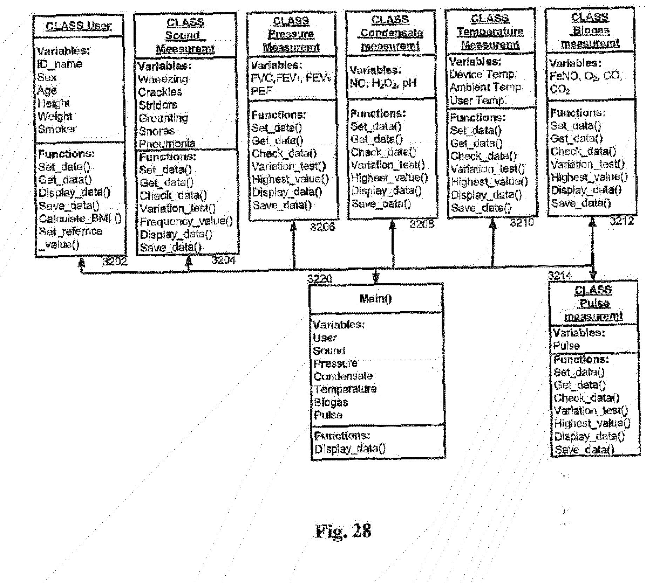

Fig. 28    Shows the different classes of the input variables for the neural network. It is identical to fig. 10 but with more details.

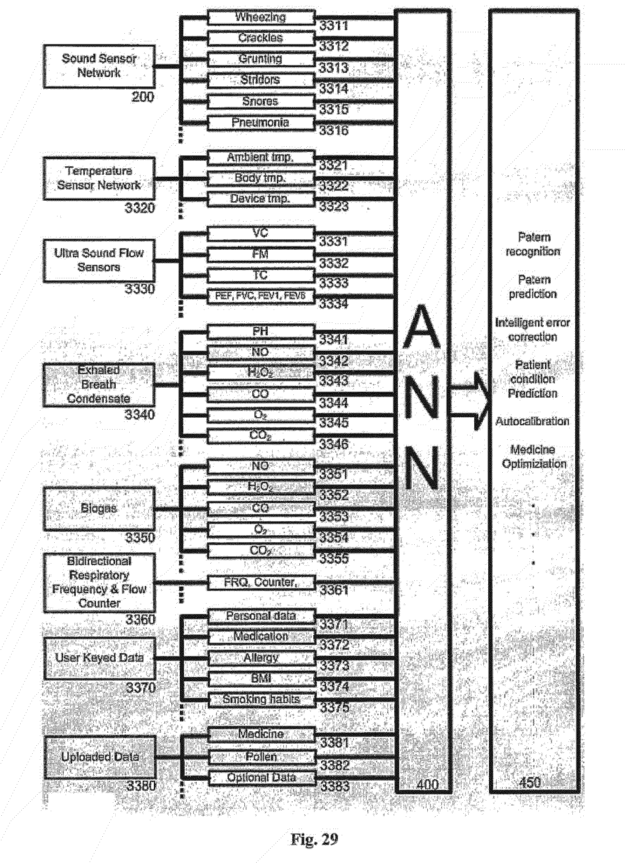

Fig. 29    Shows the different groups of the input parameters and data to the artificial neural network and the output of the same network for pattern recognition behavior. This fig. is identical to fig. 13 but with more details.

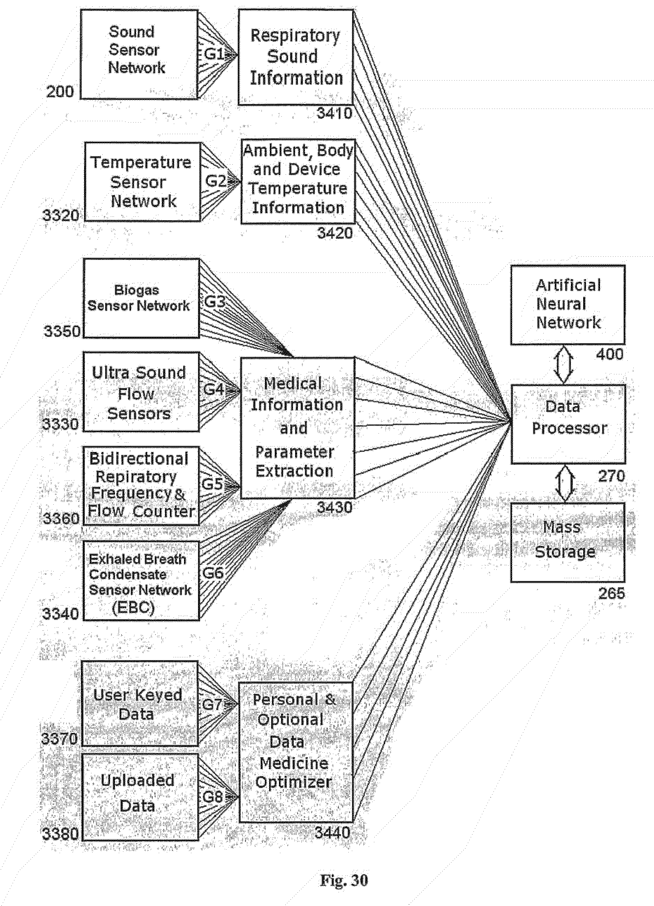

Fig. 30    Shows the interconnections of different system blocks. This fig. is identical to fig. 15 but with more details.

## DETAILED DESCRIPTION

The term "device" or "the device" or "main device" refers generally to the mother device, otherwise another term mentioned.

The expression "a given" medical term or "a given" technical term means/refers to a given mathematical calculation, a given algorithm, a given technique, a given principal a given process, a given progress, a given condition, a given diseases and/or two or any combination of them.

The expression "a given" as a general written term means simply a different nuance and/or variations of expression itself.

The term "User" refers to a patient, person, adult, children, sportsmen's and/or living organism as well as healthy and ailing person.

The term "Doctor" refers to a medical Doctor, medical specialist, Physician, Hospital, medical clinic or the like, whom are able to understand, analyze, tread and make decision to a given medical circumstance, condition, diseases, tread medical information or the like.

The term "sound" refers to a respiratory sound generated by a human or a living organism, which can be generated in the process of normal respiratory process by for example a regular healthy user or a sportsman, or abnormal respiratory process, caused by asthma, bronchitis, allergy, COPD (Chronic Obstructive Pulmonary Disease), physical lung reduction or other lung functionality disturbances. The sound can be generated through sleep, under narcosis, coma or consciousness. By the "sound" here and in the future is meant wheezing, crackles, snores, striders and the like.

The term "Sound Sensor Network" or "SSN" refers to a network of few or several sound sensors, that are couple together in a network, which can consist of parallel, series, differential, summation, subtraction, in a matrix form, for example in case of noise compensation to collect the environmental or ambient sounds as a common mode signals and the sound information as differential signals, or the like coupled sound sensors or a combination of such coupling. According to those principles, some of SSN can be coupled differentially and the other common mode.

The term "Artificial Neural Network" or "ANN" or "NN" refers to an intelligence algorithm or unit consist of a software algorithm, implemented hardware unit or a combination of both them or the like and it use to be trained, learned, predict, recognize a pattern or the like.

The term "recognize a pattern" or "pattern recognition" refers to training and/or learning an Artificial Neural Network to be able to recognize a specific medical condition, medical circumstance, diseases, medical tread, a healthy human or sportsmen's parameter, general human behavior and/or medical parameter to optimize and achieve more accuracy results, diagnosis, measurements and/or calibration or auto calibration of the Sound Sensor network.

The term "cylindrical" or "cylinder" or "tube" or "pipe" refers to any 3 dimension (3D) any oblong airflow tube/pipe such as cylinder, oblong oval, conical, square, conical and/or rectangular shaped or other 3D oblong formed respiratory pipe. Figure 2 and Figure 14 illustrates three different examples.

The device consists of one mother device (1) or main device acts as stand alone device, which can also acts as base station for one or two small ear devices (210 and 211), which should be placed in auditory meatus/auditory canal. All 3 devices communicate bidirectional and interchangeable to together (555).

The sound sensor network device Fig. 2 has a cylindrical figure 14A, (1401) or conical figure 14B, (1402) shape and can be made with/without a lay of sound isolation material like (12) in figure 2. The device has a respiratory port in the front Fig. 2-2 and a ventilation port Fig. 2-11 at the end.

The device has a modular design, which means that it is possible to couple and decouple additional Sound Sensor modules onto the body of the tube for example Fig. 2-10 shows the module location that will be used for additional Sound Sensor(s) modules and thereby improving and expanding the collection of physiological information.

All Sound Sensors will be in constant communication with the whole Sound Sensor housing through an information data bus Fig.1-262, and additional Sound Sensor information can simply be obtained by clicking more sensors to the basic housing through the embedded interface plug.

For example the respiratory sound information can be integrated with non-respiratory information collected by other type of sensors connected to the Sound Sensor network device through the sensor network data bus structure in the device for example Bidirectional Air Flow Detector (BAFD), Flow Meter (FM), Biogas Sensors (BS), Temperature Sensors (TS), etc..

In the interior of the cylindrical, conical, square shaped or other 3D oblong formed blow pipe Fig. 2 and Fig. 14, the sound sensor devise is build around a Sound Sensor Network (SSN) and is composed of a system, method and a neural network building upon the human or living organism's behavior.

SSN in Fig. 1B (200) and SSN in ear devices fig. 1A (210) and (211) built around a system, consisting of one main device (1) and two ear devices Fig. 5. The main device (1) acts as base station. The main device fig. 1A and fig. 1B can be connected through a wired or wireless (510 and 555) connection to ear devices Fig. 5. All three devices communicate bidirectional and interchangeable together through for example an electromagnetic wave (555) or a wired link for example a cable, which is not shown.

Fig. 1C shows the coupling between the sensor network "the cylinder" and data processor unit and its peripheral units figure 1B. Block (199) in figure 1C illustrate an amplifier, which is matched to it's respectively sensor. These amplifier are not shown in figure 1B and ear devices figures 5A and 5B.

The main device Fig. 1A (1) is able to be connected to ear devices (210) and/or (211) or disconnected both of them, which is now acting as stand alone and all in one device figure 1A (1). There are 5 different modes that mother device can be connected/mapped together to the ear devices. The mother device figure 1A (1) acts as stand alone device, the mother device is connected to both ear devices (210) and (211), the mother device is connected to the right ear devices (210) or the left ear device (211). The last mode is the connection between main device and ear device (210) and/or (211) regardless of the position of the ear device(s), i.e. no registration of light and/or left position, regardless of there is single ear device or both of them.

The main device can distinguish between which ear device has been mapped to the right device (210) or the left device (211) and the positioning of the ear devices should be keyed by the user or find automatically by main device. That's why two different setup modes can be installed by a user or a doctor to the main device for this purpose.

By ear device fig. 5A and figure 5B is meant a device, consisting of Sound Sensor Network (some of 224) and other sensor network, such as Temperature Sensors (some of 224) and/or Pressure sensors (some of 224) and/or Moisture Sensor (some of 224) and/or Sound Reproducer (not shown) for example a speaker, which should be placed in auditory meatus/auditory canal or earpiece. After collecting, mapping or handshaking between the devices, the information from ear device will be amplified, digitalized and afterward sent to the mother device Fig.1A through a wired or a wireless connection. The ear device SSN collect lung sounds and those respiratory sounds are used for compensation environmental or better Signal to Noise Ratio in a given measurements under condition of noisy environmental or simply for achieving more precision on a measurement for example in case of clinical use with more accuracy requirement.

The ear devices 210 and 211 are identical except the position and the mapping algorithm to main device, which provide that they be identified as right and left device.

The Sound reproducer can for example signalize a user an error under measurement procedure in a form of for example displaying through a miniature microphone an error message or the like. The user has an option setup to communicate/signalize to the device with help of specking or simply by pressing/changing a pressure through the Eustachian tube, which will act as action or confirmation or the like. The speaking can be understood by ear device(s) without any need of taking the ear device out of the ear.

The mother device can also ask, through a sound reproducer or digital display or the like, the user to place and keep one or both ear device(s) to a specific area of the body. These body areas can be shown by a doctor or a user manual of the device. These body areas can for instance be around heart, wrist, lungs stomach, neck, throat or the like.

The ear device coupled to the mother device together can have an optional functionality as a radio receiver, transmitter and/or transceiver and the like and/or music player for instance MP3 or the like.

The information through total sensor network are collected from a user through a mouthpiece device Fig. 1A (2) and further through a pipe Fig. 2, fields located directly or by distance after mouthpiece (2) and filter (3).

the ear devices. The ear devices are a miniature device the main device.

The sensor network figure 2, 3, 4, 5 and 8 consisting of sound sensor network 5, 6, 7 and/or 810 or the like, and other sensor network, can be placed at (5, 6 and, 7) in Fig. 2 and/or Sensor network in figure 8, (801, 802, 140 and 143) can have flat or 3D form and can be Mounted Surface Micro- or nanotechnology sensors. Fig.8 shows a longitude plus (+) formed sensor network that can have any flat (-) or 3D formed shape for example a longitude plus formed spiral or the similar shape.

The data is stored to the Mass Storage Unit 940, FIG. 9A and 9B, through data bus 262. Mass Storage Unit A, shown as block 910 is a read only stores and include all input data with date and time.

Mass Storage Unit B, shown as block 920, is used to store the learned information and the stored information is treated in ANN block 960. Blocks 911-913, 921-923, 961-963 shows every single unit of 910, 920 and 960. The amount of those units varies and FIG. 9A as well as FIG. 9B have only illustrative purpose to be used as an example.

The device is able to establish communication to an external data processor through a wireless (510) or a wired (not shown) link such as USB, Bluetooth, Infrared and etc., similarly transmit and receive data to and from the data processor and its result be stored into a memory or database via a software or hardware interface and/or both locally or an internet server.

The collected information can be shown as real time curves on a display, monitor, or other type of displays or an external device for example PC, laptop, PDA or the like or printer printing a curve through a wireless or wired link figure 1 (510).

The system and methods are flexible in its design and will be independent upon the type of disease. This means that the system can be adapted for several types of measurements, just by changing the loaded software with respect to which (the changeable) sensor network module (5, 6, 7 and 810) are in use.

The apparatus is capable of allowing a given user access to an automated process for managing a specified health problem, disease, better life condition and earlier prevention of disease. These benefits are obtained primary by storing and analyzing respiratory sound information through the Sound Sensor Network and the Artificial Intelligence data processing.

The analysis of the sound is based on the idea that the respiratory sounds, produced by one are unique and could be used for asthmatic and allergic sicknesses diagnosing. That makes the usage of sound analysis reasonable for the purpose of the personalization of the device. The respiratory sounds, produced by a user, should be registered in both time and frequency domain and stored to be used for sickness' diagnosing, for creating user's condition history, which could be accessed in the form of data tables and curves and later for user's condition prediction.

Further the system and method relates to automated self measurement and analyzing of respiratory sound information from living organisms.

Another aspect is directed to allowing a user access to an automated process for managing a specified health problem called a disease.

The related system and the method for its use are used for imaging and/or guidance and/or diagnosing and/or controlling purposes and are capable of communicating with external data processors for example PCs, Laptops, PDAs, Mobile Phones, etc. through a wired link or wireless connection.

More particular the combined sensor modules, system and its methods of use relates to a personal handheld home monitoring device, being an intelligent portable system and methods related to specific health issues.

Furthermore is the device generally capable of being used as a medical journal, data acquisition, storage of measurements, medicine optimizer, monitoring, night monitoring, night surveillance, storage of keyed and/or calibrated personal input data and user health behaviors, as well as short time or long term data acquisition, storage and analyses.

The functionality of the apparatus could be described as following routine. When the device is turned on for the first time or the device's master reset is activated, the device will run the initial routine, part of this routine shown as an example in figure 7A. Under this progress a user will be guided through the instruction to answer a lot of questions, which will be displayed on the display, played by a media from the device or from a display and/or media that is coupled to the device for this purpose.

After calibration of the all sensors and its network constellation and start-up mode 793, personal data 795, relevant health information 797 and information about medicine 799 should be entered, displayed sequentially in 801, 803 and 805 corrected/confirmed by running the correction algorithm if necessary in 807, 809 and 811 and saved and stored in 813, 815 and 817. The subroutine will ends in 819. Personal data and health/medicine information could contain ID (name and number), sex, age, height, weight, smoking habits, illness, usage/dosage of different medicine, additional keyed/uploaded data and optional data, information from the doctor or hospital, allergy and pal, etc.

All these input information/data will be stored in the device. The device will use this information to calculate the specific person's (user's or user's) medical, physiological and other parameters, standard values or reference values such as Body-Mass Index (BMI), PEF, $FEV_1$, $FEV_6$, FVC, VC, etc. A few examples of reference/standard values has been mentioned below ((Berglund E, Birath G, Bjure J, Grimby G, Kjellman I, Sandquist I, Söderholm B. Spirometric studies in normal subjects. I. Forced expirograms in subjects between 7-70 years of age. Acta Med Scand 1963; 173: 185-192; A J Nunn, I Gregg, "New regression equations for predicting peak expiratory flow in adults" - concerning this article more than 20 references at all could be named and incl.

For girls:

$$\log_e FEV_1 = -1.5974 + (1.5016 + 0.0119*age)*height,$$

$$\log_e FVC = -1.4057 + (1.4800 + 0.0127*age)* height,$$

$$FEV_1\%FVC = 88,88$$

For boys:

$$\log_e FEV_1 = -1.2933 + (1.2669 + 0.0174 \cdot age) * height,$$

$$\log_e FVC = -1.2782 + (1.3731 + 0.0164 \cdot age) * height$$

$$FEV_1\%FVC = 86,21$$

For females:

$$FEV_1 = 1.08*(3.95*height - 0.025*age - 2.60)$$

$$FVC = 1.15*(4.43* height - 0.026*age - 2.89)$$

$$FEV_1\%FVC = 89.1 - 0.19*age$$

$$\log_e PEF = 0.376*\log(age) - 0.0120*age - (58.8/height) + 5.63$$

For males:

$$FEV_1 = 1.08*(4.30 \cdot height - 0.029*age - 2.49)$$

$$FVC = 1.10*(5.76 \cdot height - 0.026*age - 4.34)$$

$$FEV_1\%FVC = 87.2 - 0.18*age$$

$$\log_e PEF = 0{,}544^* \log_e(age) - 0{,}0151^* age - (74{,}7/height) + 5{,}48$$

This information will be stored and compared to the measured data later and stored again and again. Each of this information sets reaches a date and time stamp in the store unit.

During the time the device will achieve more information about the specific user through the measurement results and through commutation between the user and the device. It will result to that the device will achieve more accurate information about the user, therefore the reference or standard values will be not used anymore, these values will be still on the device storage unit. The user has always access to all this information, but the user is not allowed to change, delete or overwrite the data once they are stored, thus the information are a port of the Artificial Neural Network (ANN) resources. Although ANN has an adaptive character, the data collected will be saved in the device and could be accessed later in order to review user's condition. After a given period of the time, when the device will learn more about the user, and a set of user's outcoming data will be collected, compiled and stored as input data for the ANN part, each of this input data will get a set of date and time stamp.

When a measurement procedure is activated or if a timer activated and set up to periodic alarm indication to remind the user to perform a given measurements, the device will run a program and algorithm internally and afterwards the user will be guided throughout to take a (or several) measurement(s). The alarm will indicate the measurement event by a indication of a text on display, sound or voice from the speaker, light a LED, shake alarm and the like.

So the measurement will begin and the program will run. An example of a routine is illustrated by figure 7B (main routine) blocks 701 to 735 and figure 7C (subroutine), blocks 737 to 755. In this particular example figure 7B, the system starts with block 701 and request for deep breathing program. THE DEVICE goes into measurement mode 703, where subroutine 7.2 block 737 is called. The type of the measurement mode is classified in 739, and the test begins with internally defined Flow Meter (FM) mode, where all measurements are taken in the process of not forced inhalation and exhalation. There registers temperature ° C, FMin (inhalation parameter) air flow, FMout (exhalation parameter) air flow, lung sounds (wheezing, crackles, snores, etc.), $FEV_1$, $FEV_6$ and FVC in the block 745. In the mode of forced inhalation and exhalation 747 PEF, $FEV_1$, $FEV_6$ and FVC are registered. If the measurement is a PEF type, the user is asked to perform a maximal expiration 3 times each followed by a rest period (usually 1 minute).

The PEF value is calculated in 745 for the 3 tests and the values are checked for some reliability criteria's in 749. If the criteria's are passed the value(s) are stored in the memory, but if they fail the user is asked to redo the measurement in 753. The PEF measurements are usually done 3 times with 1-minute intervals between the measurements.

If the measurement is of the second type, the user is asked to perform a maximal forced expiration in 743 minimum 3 times. The values are also checked for some reliability criteria's in 751. If the criteria's are passed the value(s) are stored in the memory in 753, but if they fail the user is asked to redo the measurement in 753.

All the $FEV_1$, $FEV_6$ and FVC values are then calculated and stored in the memory.

The essential part of this procedure is that it can minimize the measurement errors caused by wrong used of the device when used for home measurements. This minimization of measurement errors is achieved by requiring the measurements to be reproduced with high accuracy.

The measurements flowchart are only illustrative, many different types of measurements can be performed in conjunction with this device.

Comparison of the measurements with standard reference values 710 starts in 783 subroutine in figure 7E. After loading data in 783, calculation the percentage deviation 787 and setting indications flags containing the percentage deviation for each of the measurements 789 are performed and end in 791 .

$FEV_1$/FVC ratio 715 calculation by formula ($FEV_1$/FVC) ·100%, performed in order to set asthma/COPD diagnosis. Please notice, that the apparatus is not setting the medical diagnosis, but the diagnosis, set by a doctor could be based on the date collected and analyzed by the apparatus. If there are found any signs of illness in 720 (more than 70% for a healthy human), indications flags 723 and 717 are set. These flags can then be used by the physician in his overall diagnose of the user of the device. After loading PEF measurements in 725, day per day variation 727 is calculated by formula $PEF_{max}$ - $PEF_{min}$/$PEF_{max}$ x 100 %. The percentage deviation 731 defines indications flags 729 and 733. The procedure ends in 735.

A simple realization of a display can be a digital display, LCD on the device self or a display that is coupled to the device for this purpose such as mobile phone, PDA, Palm, Laptop, PC or the like through a wired or wireless connection. A simple realization of spoken media can be a simple speaker that is mounted in the device self or a spoken media that is coupled to the device for this purpose such as mobile phone, headset, hearing aid, PDA, Palm, Laptop, PC or the like through a wired or wireless connection. There is possible to encrypt this information to protect user's personal data and information, this facility is not shown.

The intelligent part of THE DEVICE is based on Artificial Neural Network (ANN). ANN takes as input the data measured and is trained to recognize the specific user behavior, a given pattern as mentioned below, what makes THE DEVICE highly personalized. It includes also sound analysis 7.5.

Lung sounds produced by a user are unique and as following could be used for COPD/asthma behavior prediction. When the user has trouble breathing because of asthma, the problem is in the airways of the user lungs. The airways become narrow because the muscles around them tighten, their inner linings swell, and extra mucus clogs smaller airways. Breathing gets harder as user try to force air through the narrower airways. The air the user breathes may make a wheezing or whistling sound.

After the end of learning period the device should be able to know more about the specific user's pattern behavior, more accurate data etc. as mentioned before. Thus the device should be capable to estimate, calculate, recognize, calibrate and correlate exactly the value of different measurable parameter for user's condition and should be able to correlate for the measurements value by analysis of lung sound and few other parameters and calculation and estimate other washable measurement values.

The use of Artificial Neural Network (ANN) in Software Development and its goal setting. When the Device, figure 1A, 1B and 1C, turned on, the device will goes automatically into self-test mode Figure 7A and making initializing processes, test & calibration of the sensor network and checking the data. Now the device goes automatically into Flow Meter Mode (FMM), and ask user to breath normally through the mouthpiece until device indicate for second phase of measurement. Afterwards the device indicates for second measurement, which the user should breathe deep through the device, like when a doctor listening a patient's lung with a stethoscope. Under these measurements the user should be sitting, standing or lying relaxed and breathing normally, e.g. without forcing.

The device will measure user's bidirectional inspirations and expirations and afterwards will measure, calculate and store different important parameters such as lung sounds (both in time domain and frequency domain), vital capacity (VC), temperature of lungs (see below), etc. These parameters will be measured, registered, checked and saved. The same should be done when THE DEVICE goes into measurement mode (FVC, FEV, etc.), performed on the condition of forced breathing.

User's unique and personal parameters will be learned and stored by a learning progress, through the devices' Artificial Neural Network module. The algorithm should remember given patterns to determine user's condition when he is relatively healthy ("normal" condition) and relatively sick ("abnormal" condition). For every FM mode THE DEVICE with help of the ANN should guess user's condition and after completing measurement mode THE DEVICE should compare the data guessed with the data measured practically. The idea is that when this comparison will give error percentage less than 2%, the device will be able to predict user's condition, e.g. COPD/asthma behavior, every time it will go into FM mode. This will help a user to prepare himself for sicknesses "peaks" and "quiet" periods as well as to administrate own medication. Another purpose is to remember user's data patterns in order to precise the measurements. Existing apparatuses are usually 90% precise, when THE DEVICE with help of ANN should be closer to about 98% precise. This will help to diagnose a user more carefully as well as administrate medicine doses with higher precision.

There are 6 groups G1-G6, fig.13 of input should create data patterns for ANN learning/training and further pattern recognition. Those data patterns will have a form of time series figures 9, 10, 11, 12, 13 and 15.

Furthermore, those time series could be treated as input files for ANN, using back propagation (general solution), fuzzy logic, simple linear ANN or other types of network. The possible structure of the desirable functionality is shown on figures 9, 10, 11, 12, 13 and 15. Some of input parameters are shown on figure 13 and should be not taken in a limiting sense as they are only illustrative - much more parameters could be deployed in the later versions.

The displayed or spoken instruction for example the speaker should be answered by the user, doctor, hospital staff, related or other through the keypad, dictated command or other form for indication. This information, the answers and the input data etc. can also be uploaded to the device by the user self or a doctor etc. via a wired or wireless connection 510.

The classifier of the information is achieved through the commutation between the user and device, these classifications such as user's physical condition, psychological condition, allergy specification and the degree, headache and its interaction, pulmonary condition, respiratory condition, mood, "low spirits", happiness or other related pattern, that is important for user. Users behavior itself or users reaction on an out side word parameter such as weather, a given condition, house-dust mite, different smoke or gas, food or drink/liquid and the like and its interaction.

In the optional part of the device, the user capable to set the device up to learn, recognize, optimize a given condition, parameter, behavior, pattern for a medical, medicine, healthy, pollen other parameter which is the user wishes to learn more abut or administrate.

After setup of the device for first time or after master reset activation and a new setup setting, there is possible for a doctor/specialist to choose a specific input data/parameter to not be taken in account for optimization in the ANN training progress and classification. Because of for example if in a given country is not allowed a given parameter to be optimized in ANN area. Similarly it is possible for a physician/a doctor or specialist to choose a specific parameter to be taken in account for optimization in the ANN training progress, but for a given specific period for example 14 days, a month or the like for example for achieving more accuracy or better calibration of device. The end of this period will the device not be able to any more optimize the specific parameter further.

The term "User" shall refer to a patient, person, adult, child, sports person and/or living organism as well as healthy and ailing person.

The lung(s) sound can be generated during sleep, under narcosis, coma or during unconsciousness. The term "sound" here and in the following is used to denote wheezing, crackles, snores, grunting ,striders and the like or the like and shall also refer to other sensor networks not related to sound.

The expression "biogas" shall refer to all exhaled gasses, atmospheric gasses, and other gasses or vapors relevant to asthma patients such as NO, $H_2O_2$, $O_2$, $CO_2$, CO etc.

The expression "humidity" shall refer to humidity in general terms, the humidity or vapor in the atmosphere, and exhaled gasses exiting the user's body e.g. from the mouth, the nose and the ears. These gasses comprise liquid particles e.g. $H_2O$ and $H_2O_2$ particles and the like.

Whether mother device is paired to the right ear device (110) or the left ear device (111) and the positioning of the ear devices can be keyed in by the user or be found automatically by the main device. That is why for this purpose two different setup modes can be installed by a user or a doctor to the main device Ear device figure 1 blocks 110 and 111 shall refer to a device, consisting of a Sound Sensor Network (SSN) and other sensor networks. These sensor networks can be a combination of any of the following according to the requirements of the observation:

1. Temperature sensors
2. Pressure and flow sensors
3. Moisture sensors
4. Sound and vibration sensors
5. Biogas, biomedical and/or electrochemical
6. optional detectors

In addition hereto, a sound reproduction device e.g. a speaker is placed in the auditory canal (meahus) or earpiece.

In addition the device may also be used for language training. The device can be used when working, resting or sleeping.

Since the mother device has a large/ extensive memory capacity, and communicates with the ear devices, it can also be used to transmit other kinds of information to the ear such as recorded music. Mobile phones connected to the mother device can therefore also be operated through the ear device. Optionally the mother device can have a built-in radio receiver

Figure 1 (102) and further through a tube (or pipe) Fig. 2 filter is located directly or behind the mouthpiece. The ear devices are miniature versions of the main device and/or are identical with the mother device regarding

The ear devices are a miniature version of the mother device and are internally identical with it. The key pad and display functions of the main device are emulated by the speaker and microphone components of the ear device. This enables the ear devices to operate and communicate through voice commands.

The ear device or devices can work as a stand-alone device/implant device and other device as mother device for instance a PC or laptop of PDA or the like and perform the same functionality by loading it's program.

Figure 1C shows the coupling between the sensor network cylinder and data processor unit and its peripheral units.

Block (199) in figure 1C illustrate an amplifier, which is matched to it's respective sensor. These amplifiers are not shown in figure 4 for the ear devices. But ear devices are a miniature equivalent of the main device. The block 199 and 2130 in figure 21 generally have been used throughout the text to indicate an amplifier or preamplifier for different amplification purposes , blocks or sensors. These blocks 199 and 2130 indicate a symbol just for amplification of the particular signal but the technical data of each of these amplifiers are not specified.

The device is capable of allowing a given user access to an automated process for managing a specified health problem, disease, healthy or better life condition and earlier prevention of disease. These benefits are obtained primarily by storing and analyzing respiratory sound information through the sound sensor network and the Artificial Intelligence data processing.

These could be accessed in the form of data tables and curves and later be used for predicting a user's condition.

The device is a managing and advising proposes and not a medical diagnosis without a doctor, but the diagnosis, set by a doctor could be based on the data collected and analyzed by the device

There are 8 groups G1-G8, fig. 15 and 30 of measurements and store of data input. These data inputs are basic to the learning process of the ANN and its pattern recognition figure 13 and 29, and earlier prevention, better life condition, etc. managing health and disease conditions. Those data patterns will have a form of time series figures 6, 11 and 12.

In one embodiment of the invention, the sensor network is placed on the internal sidewalls of the sensor modules inside the device Fig. 3 and 14, which forms a three-dimensional array of sensors.

In another embodiment, the sensor network is placed in the middle of the device, forming a net. Fig. 2B and 2C are examples of such a network of sensors.

In a third embodiment of the invention, a combination of the first and the second embodiments are use together.

Fabrication example for biogas- and biochemical sensors such as NO sensor, the following sensors can with small modifications be used for example ISO-NOPMC from company WPI, industrial NO-sensor Type I-25 from International Technologies Dr. Gambert GmbH and amiNO-700 or amiNO-100(size 100 micron) from company Innovative Instruments Inc., or planar sensor array shown in fig. 23. These three companies also have other types of biochemical /electrochemical sensors such as pH, H2O2, CO, O2, CO2 etc.

All units are powered up by battery, power supply fig. 1B block 500 or implemented simply by a single SoC solution VLSI-like structure.

The ear device or implanted device can be realized like a miniature model of the mother device except for the LCD display and the keypad, for example made of single module system-on-chip (SoC), single ASIC, the micro-sensor network fig. 23, lab on-a-chip fig. 23, memory, sensors network and sound-sensor-transceiver fig. 21, fig. 5A block 220. The ear device can be connected or paired to a data processing machine via a wireless or wired link. The ear device can automatically transfer the data to data processing machine when it is in its socket or the base and charging station fig. 5A block 226.

Monitor and key pad fig. 1A, blocks 286 287 interface the microprocessor fig. 1B block 270 through a user interface 280 or through an external computer or the like.

**Features/functionalities:**

The mother device and the ear devices may be identical, fig. 1A. They may therefore include the functionalities, functions and features of the other device. The mother device and the ear devices use the same measurement parameters. The device can be fabricated in such a way that comprising one or more measurements and functions at the same time, a given relevant combination hereof, a quite new measurement/function and/or all of them included in the application or a combination of them. Ear device or mother device can act as stand-alone-device is fully functioning and measure a few parameters.

**Snoring disorder:**

Two scenarios: inconvenience due to disrupt sleeping or actual disease.

The mother device and the ear devices may be identical. They may therefore include the functionalities, functions and features of the other device. The mother device and the ear devices use the same parameter measurements.

As another example the sound-sensor-network-device can be set up to measure the user's sleeping snore. The device by receiving the snore-signals can afterwards digitalize and process data in a signal-processing-unit. These identified signals can then be recorded or stored in the memory of this device. After a period of use - or by activating its neural network - the device is able to recognize the snore-frequency-components of the user.

After a while all these frequencies are mapped in a pattern-recognition-behavior-unit or a table or in an array.

In the same way, the device can detect other sleep disorders like apnea.

**Language training/teaching:**

The sound sensor network device can also be used for language training/teaching. The language trainings program, data or file may be up loaded to the mother device in form of MP3 or similar formats. The user has the option to pair the ear device or devices to mother device during work, recreation or travelling.

**Water-proof:**

There are two options/possibilities: the ear devices and the mother device may be water-proof or may not be water-proof.

**Measurement of exhaled NO in exhaled breath condensate and liquid:**

For the measurement or analyzing the atmospheric gasses or exhaled biogases (for instance NO, $H_2O_2$, $CO_2$, $O_2$, or the like) and the liquid or contents of exhaled condensate, there are four different methods or more. One is to use a mechanical shape or form in the actual tube, e.g. a Gaussian surface (3D) fig. 20 block 2010, ball-shape, sphere, hemisphere or similar. The Gaussian surface or similar model can be formed in such a way so that humidity is collected on the surface after which it is gathered on the tip in the form of small liquid particles or drops fig. 20, blocks 1414 and 2010. These Gaussian surfaces can be formed in such a way so that humidity is collected on the tip or the certain area of surface, thus the device is able to decide the amount of liquid for measurement contents.

Hereafter the sensors can measure the humidity contents, e.g. NO, $H_2O_2$, pH or the like. The sensors may be positioned on the tip, on the broader surface, or it may have other positions. Fig. 20 block 1414 shows a Gaussian surface and its position in the tube.

Another method is to use a chamber. With regard to the chamber design, the air can circulate within the chamber after which humidity is collected in the lower part of the chamber. The sensors may be positioned at the lower part of the chamber or may have other positions. These chambers are formed in such a way, that humidity easily is collected in a certain area, thus enabling to decide of the amount of liquid for measurement of liquids contents.

The third option comprises a moving sensor.

The moving sensors can be formed in such a way so that the sensor rotates and collects particle samples. Moving sensors can measure biogas and humidity. Moving sensors comprise sensors network.

Non-moving as well as moving sensors can measure biogas and humidity. Some of these sensors function as reference sensors and some as measurement sensors.

In case of all of the above-mentioned sensors, a microchip, cooler-device, peltier-device or thermoelectric-device (e.g. DT33-401LS from MARLO INDUSTRIES) or similar can be mounted on the surface of the tube, on tip of Gaussian surface, in the lower part of the chamber, to generate liquid from humidity from atmospheric gases or exhaled gases. The sensor either can be positioned close to the chip or can be integrated by way of annealing, welding, be glued or similar methods.

The device can measure important fluid parameters in liquid humidity, gasses and plasma, as well as for example in condensate water. These parameters can be measured through a connected external sensor or inside the very device. Internal measurement is done by using a microchip or a mechanical item e.g. Gaussian surface catching the condensate water of the exhaled gas and analyzing its chemical contents by using a sensor. The parameters may consists of NO, $H_2O_2$, CO, pH, or other items, which can be used for diagnosing or monitoring disease, or for other kinds of analyzes.

The air flow through the tube can be forced by micro-mechanicals, MEMS, or a miniature motor with a propeller placed inside or outside the tube or device. This facility which is placed in- and/or outside the device or tube can in the same time be used to reach a constant flow through the tube, for making a uniform velocity profile or for producing a certain resistance or pressure of the airflow.

To indicate and measure the total amount of NO produced both in the nose and in the lungs the device can be inserted into one or both of the nostrils. The patient has to keep his mouth closed during this process only exhaling through the nose. The device detects both NO produced in the nose and in the lungs. Afterwards we detect the exhaled breath from the mouth and in this way we are able to compensate the error of the nose-produced NO.

When using the existing biogas and chip sensors available in the market for detections and analyzes of exhaled NO-information nearly all of them demand constant humidity and temperature and some of them even constant flow.

Surface and chamber must be significantly colder than the exhaled gasses for condensate to be produced. The changes of the temperature can for example be made by a chip.

The very Gaussian surface, the chamber and the chip solution must all meet the condition of being much colder than the exhaled gas.

The user breathes into a plastic or a metal tube in the device containing the already mentioned forms, for example Gaussian surface. These different items for gathering condensates should be surrounded by cold metal or by a thermal-electric device in such a way that vapor from water or exhalation from the lungs can be developed as condensate.

Normally it takes about ten minutes to get enough condensate, but this process can be diminished by using for example the Gaussian surface.

In some cases the device indicates when enough condensate is gathered for making a measurement. For some sensors a thin layer of steam is enough to be able to measure and to make analyzes of exhaled breath condensate.

In one embodiment of this invention it is possible to measure biogasses by measuring the condensed form of exhaled gasses.

In the performed measurements a coherence between Fractional exhaled NO in parts per billion (FePPB) and NO in exhaled breath condensate (EBC) (pA/nM) has been observed. Also a linear relationship between Picoampere (output of the NO sensor) per nanoMolar density (PA/nM) and FePPB of eNO could be demonstrated. Three different tests are made:

    1. Measurements directly on exhaled NO (figure 1A, 1C and 2).

    2. Measurements of NO in exhaled breath condensate (figures 24 and 25)

    3. Measurements have been made with the addition of enzymatic reagent to a standard buffer solution containing a known concentration of NO (figure 24 and 25). The release of NO from the water solution to gas face could then be demonstrated and reproduced repeatedly with dose dependent signals (Fig 24) All three tests have been shown to correlate this exactly measurements of exhaled NO with the accuracy of a few parts per billion. Also, the measurement could distinguish between individuals with varying degree of bronchial inflammation.

This test was repeated both with person with healthy lungs and with persons with a high amount of exhaled NO (asthmatic patients). An amount of more than 25 parts per billion or more is considered to detect asthmatic lungs or lungs with bronchial inflammation.

We have as an example tested persons between of an amount of 15 parts per billion (healthy individual) and persons with over 35 parts per billion (asthmatic individual). The microsensor chip fig. 23 is a planar realization of the electrode in fig. these micro-sensors which are unique in the properties of the sensitivity and selectivity of the sensor surface or sensor tip.

Those sensitivities are achievable party by the silver material of the electrode or planer and by the positive resting potential of 18 mV-850mV. Also, the small distance between the sensor tip and reference electrode is important properties that diminish noise from other intervening molecules. Lastly, and importantly, the selectivity is achieved by the semi permeable properties of the sheeting membrane that effectively exclude other molecules from diffusing in to the covered sensor tip, and almost only allows NO to pass. By using same principal with other material and sheeting membrane it is possible to make other sensor that select different liquids or gases. Fig. 22 and 23 shows two different examples.

By placing one or more discs fig. 17, blocks 1710, which can also be used to form a uniform velocity profile or to create resistance and pressure when blowing in the device. The smaller the pipes are through out the disc, the more resistance and pressure is produced.

The discs comprise a few or several pipes. The pipes comprise uniformed or different size or same sized; round, circular, 2D, 3D any form of longitudinal form and the like.

By placing a disc of pipes (fig 17) in the mother device a relative constant flow is achieved, and by using exhaled condensate a constant humidity can be achieved in the tube. This demands a position of the one or more valves (not shown in fig. 17) after the mouthpiece but before the position of the sensors. When the user inhales through the mouthpiece, the valves open, and let the air pass through. When the user exhales, the valves close and the air passes through the mouthpiece and then through the mother-device and are passed away through flow exit-port (examples are shown in fig. 1A and fig. 2). The opening and closing of the valves are not activated at some measurement like bidirectional flow measurements.

These parameters may for example be:

The device can have preinstalled or preloaded a certain/particular software to perform and fulfill a few or more functions for a short or a long period, for instance for 2 or 3 weeks or for one year or for a several years-program. As an example a 2 or 3 weeks program can simply consist of a measure-program for detecting user PEF-blow.

For one or several years data treatments and measurements - for example ageing of the lungs can be measured with the time dependence factor or evolution of environmental effect such as pollen, humidity and other environmental effects on the body or lungs ("user conditions") can be measured and stored.

Therefore the device and its method- and system can act as a simple machine or as a very complicated machine depending of which program the doctor chooses.

To detect snores and helping these users, the snore-detecting-device-structure can be fabricated simply without the artificial neural-network or a simple linear ANN. By simply loading the user's snore-data or using soft-ware module to map the snore-frequency in the memory and use it as a reference avoid use of ANN. There is a difference between the frequency and the acoustic of the different users. This makes the device cheaper to produce, because the expense of adding an artificial neural-network is saved.

By using this machine the device can indicate and display a warning for the user on the user's condition. The indicator or the alarm can be realized or implemented as an alarm-signal in a particular sound, speech, light or in a beep-sound of a particular frequency.

This alarm-signal can warn the user by waking him/her up or by making him/her conscious that he/she should change his/her position. The user can be taught to change their position for example from the back to a side-position. One of the combinations for the user is to wear one or both ear-devices and for the mother-device to be placed on for example a table at the bedside. The three devices all together watch and analyze the frequencies - either the ear-device or the mother-device can produce the alarm-signal to make the user turn to another position in bed.

In severe cases, for example if the user because of an illness is not able to breathe or after having been drinking alcohol, after a short while the device alarms to make the user turn to another position and the alarm-signal can also be enforced.

The same principle is used as an example for an asthma-patient and the device can again warn them before the asthmatic attack occurs.

Early warning before an asthmatic attack or before a similar condition again the device can warn the user to take their medicine or advice them some other kind of reacting depending on the degree of the condition.

The ear-device or mother device can similarly be programmed to recognize a given condition form from the subject/the machine/ or device or the like. As example the ear device or system can be programmed to indicate or monitor a particular sound or vibration. In this case the ear device can transmit this information to the mother device or to the controlling system or the like for the indication of an error, for a disorder or the like or in the same way for acceptance and improvement of the condition (production service).

### Detection of vibration and pulse-rate:

The ear device can measure and collect both vibrations and sound signals and since the heartbeat and heart vibrations are far lower than the sounds of the lungs, the device is able to detect both lung-sounds and heart-rate and make analysis to indicate the users' condition. By putting the ear device(s) on the artery of the wrist, on the heart or on the artery of the neck you can also measure heart-signals and pulse-rate fig 5B.

### Connection of the mother and/or ear device to hospital-equipments:

There is possible of entering the mouthpiece of the mother device as a link between the mouth of the patient/or a mask and a hospital-equipment (such as respirator) to enhance hospital-equipment with more parameter and detail analyses, supervision, alarm a given condition and data processing and the like.

### Combination of sensor networks:

In order to limit the number of devices required by e.g. a person suffering from lung desires, impaired hearing etc. The sound sensor network device can also function as a cell phone and/or hearing aid use.
To be added to "the section about loudspeakers and microphones in ear devices":
When a person suffers from impaired hearing, such person can activate an option in the sound sensor network device which means that one or more of the sound sensors is activated to capture external sound and reproduce the sound by a speaker. The sensors can be adapted to meet the requirements of the user in question fig.. The ear device can be paired to the mother device and be set upped to receive environmental sound information signals to enhance the signals and prepare them to reproduce those signals meeting the requirements of the impaired hearing of the user in question. By placing the mother device close to the sound information source signal to transmit the information to the ear device wirelessly and reproduce them for the users ear demand fig. 1A and 5A.

A simple fabrication suggestion could be described as following. The Sound Sensor Network is a network of sound microsensors, a network of micromechanical sound sensors and/or MEMS, a network of sound transducers, a network of semiconductor sound microsensors or microchips, or simply these sound microsensors produced of semiconductor material on the same substrate or silicon on insulation (SOI) like substrate. Sound microsensors can be built up through Micro- or Nanotechnology based semiconductor or other like material. The Sound Sensor Network is placed on the internal sidewall of the device fig. 2 (143), a 3D row of membranes/diaphragm fig. 2 (5, 6 and 7) and/or. These membranes or diaphragm 5, 6, 7 are building of net formed in such way, that airflow and sound can pass through the cylinder tube without any undesirable blocking for airflow or sound, which can cause turbulent, reflection or distortion of such signal information, similarly for figures 8 and 14A and B.
The Sound Sensor Network's output or response to the system include noise that caused by turbulent between the airflow and the sound microsensors 143, an algorithm and/or calculation will take this noise into account and eliminate it, for example by letting the system understand it as common mode signal or/and sound noises in compare to a differential signal information.

Similarly, if the Sound Sensor Network's output or response to the system including noise that caused by turbulent between the airflow and the sound microsensors 5, 6, 7 (chain of microsensors on membranes or diaphragm or net 5, 6, 7), an algorithm and/or calculation will take these noise into account and eliminate it, for example by let the system understand it as common mode signal or/and sound noises. These turbulent effects can also be eliminated by using flat microsensors combined with a cylinder or cone formed longitude tube as shown in fig. 14A and B, which is an alternative to figure 2.
The device can communicate with user through a display and key pad Fig. 1A and 1B. The display is a given monitor such as LCD and the like, which is placed on the device 287 in figure 1A or instead of display can the device be connected to a laptop or PC like screen through a wired or a wireless connection.
Similarly the key pad 286 is a simple key board or a few bottoms, which is placed on the device or instead of key pad can the device be connected to a laptop or PC like keyboard, which is placed on device or instead of keypad can the device be connected to a laptop or PC like keyboard through a wired or a wireless connection.

The acquisitions data or the collected data from analog micro sensors will be digitalized using an analog to digital conversion by a given discrete Integrated Circuit (IC). Afterward by using a microprocessor 270 such as Intel StrongARM SA-1100 or Intel XScale PXA255 or other data processor from Intel ® Corporation or Athlon TM Corporation or the like, which the signals can be analyzed in their in time domain and/or frequency spectrum by using for example can Discrete Fourier Transformation, Fast Fourier Transformation (FFT) or other similar algorithm be applied. Throughout this document, it will be named simply Fast Fourier Transformation (FFT) 245 as general expression. Components for the external memory 260, 261 and 265 communicate with the microprocessor 270 through a databus 261, or separately with the data processor. The chosen microprocessors have an integrated databus controller. A choice for the RAM module 260 could be Intel 28F128J3, Intel 28F128K3 or the like. The ROM module could be Atmel 28LV010 or the like. The mass storage module could be compact flash card from Dane-elec DA-CF-1024 or disc drive as Seagate St380011A and the like. Intel Xscale PXA255 has a Bluetooth™ and USB modules which could be used as wireless and wired communication 510. All units are powered up by battery power supply 500. Monitor and key pad 285 interface the microprocessor 270 through a user interface 280 or through an external computer or the like.

The data from collected from SSN or data microsensors network, which is needed to be transformed into the frequency domain through for example 512-point FFTs, 1024-point FFTs, 2048-point FFTs or the like. A simple solution is to make phase shifted for the FFT results and sum them in a frequency domain beamformer to calculate signal energies. Afterward the magnitude of signal energies can be calculated. These data will be recorded in the memory.

The Flow Meter (FM) can be implemented by using an ultrasound principal in cylindrical flow meter, which is known as Capacitive Micromachined Ultrasonic Transducers or simply as cMUT Technology. This method is an very accurate measurement.

FIG. 10 illustrates a possible idea to THE DEVICE's software, It has only an illustrative purpose and should be used only as an example of the possible implementation idea.

The design should be object-oriented. There are no comments to variables and functions because they are simple and - most important - will be surely modified and updated in the process of real implementation. By "real implementation" is meant a process of the real software development.

Artificial Neural Network (ANN) commercial fixed package example:

One of the following mentioned simulator environment and their ANN package solution, other similar products or a combination of them can be applied to development and implementation of the ANN in the device and for it uses:

1- BrainMaker
Name: BrainMaker, BrainMaker Pro
Company: California Scientific Software
Address: 10024 Newtown rd, Nevada City,
CA, 95959 USA

2- SAS Enterprise Miner Software
Name: SAS Enterprise Miner Software
Company: SAS Institute, Inc.
Address: SAS Campus Drive
Cary, NC 27513
USA

3- NeuralWorks
Name: NeuralWorks Professional II Plus (from NeuralWare)
Company: NeuralWare Inc.
Adress: RIDC Park West
202 Park West Drive
Pittsburgh, PA 15275

4- MATLAB Neural Network Toolbox
The Mathworks Inc.
3 Apple Hill Drive
Natck, MA 01760

5- Propagator
Contact: ARD Corporation,
9151 Rumsey Road,
Columbia, MD 21045,
USA

6- NeuroForecaster
Contact: Accel Infotech (S)
Pte Ltd; 648 Geylang Road;
Republic of Singapore 1438;

7- Products of NESTOR, Inc.
530 Fifth Avenue;
New York, NY 10036;
USA;

8- Ward Systems Group (NeuroShell, etc.)
Ward Systems Group, Inc.
Address: Executive Park West
5 Hillcrest Drive
Frederick, MD 21702
USA

9- Neuralyst
Company: Cheshire Engineering Corporation;
Address: 650 Sierra Madre Villa, Suite 201,
Pasedena CA 91107;

10- NeuFuz4
2900 Semiconductor Drive
Santa Clara, CA, 95052
USA

Clauses

1. A device for obtaining a physiological parameter, said device comprising:

- a housing defining a conduit having an inlet and an outlet,
- a sensor for determining said physiological parameter, said sensor being mounted in or on said conduit for generating an electrical signal representing said physiological parameter based on airflow generated by a person blowing in said conduit at said inlet, said sensor having an electrical output for outputting said electrical signal,
- an analogue-to-digital converter having a converter input and a converter output, said converter input being electrically connected to said electrical output of said sensor,
- a neural network processor having a first and a second digital input and a first digital output, said first digital input of said neural network processor being electrically connected to said converter output for receiving said signal,
- a memory unit having a digital input and a digital output, said digital output of said memory unit connected to said second digital input of said neural network processor for transmitting previously stored reference values each representing a health state of a person and identified in a hierarchy of said reference values, said first digital output of said neural network processor being electrically connected to said digital input of said memory unit, and
said neural network processor establishing on the basis of said reference values a health state of said person and presenting information relating to said health state to said person and storing said health state in said hierarchy.

2. The device according to clause1, wherein said device further comprises a transceiver for transmitting and receiving data representing physiological information determined by external units.

3. The device according to clause 2, wherein said transceiver is a wireless device or in the alternative a wired device.

4. The device according to any of the clauses 1-3, wherein at least one of said at least one sensors are sound sensors or alternatively an array of sound sensors.

5. The device according to clause 4, wherein at least one of said sound sensors or alternatively an array of sound sensors measure ultra sound.

6. The device according to any of the clauses 1-5, wherein at least one of said sensors is a temperature sensor or an air pressure sensor or a moisture sensor.

7. The device according to any of the clauses 2-6, wherein said external device is an ear piece.

8. The device according to any of the clauses 2-6, wherein said external device is a mouth piece.

9. A method for obtaining a physiological parameter regarding an individual using a device comprising a housing defining a conduit having an inlet and an outlet,
a sensor for determining said physiological parameter, said sensor being mounted in or on said conduit for generating an electrical signal representing said physiological parameter based on airflow generated by a person blowing in said conduit at said inlet, said sensor having an electrical output for outputting said electrical signal,
an analogue-to-digital converter having a converter input and a converter output, said converter input being electrically connected to said electrical output of said sensor,
a neural network processor having a first and a second digital input and a first digital output, said first digital input of said neural network processor being electrically connected to said converter output for receiving said signal, and
a memory unit having a digital input and a digital output, said digital output of said memory unit connected to said second digital input of said neural network processor for transmitting previously stored reference values each representing a health state of a person and identified in a hierarchy of said reference values, said first digital output of said neural network processor being electrically connected to said digital input of said memory unit,
said method comprising the steps of:

said individual blowing air through said at least one opening,
recording data from said at least one sensor,
storing said data from each of said at least one sensors in said memory unit alternatively in said neural network processor,
said neural network processor establishing on the basis of said reference values a health state of said person and presenting information relating to said health state to said person and storing said health state in said hierarchy.

10. The method according to clause 9, wherein said physiological parameter is peak expiratory flow rate and/or forced expiratory volume and/or forced volume capacity.

11. The method according to clause 9 or 10, said method further comprising: placing an external device on the body of said individual.

12. The method according to clause 11, wherein said external device is placed in at least one ear of said individual and/or said external device is placed on the torso of said individual alternatively at another part of the body of said individual.

## Claims

1. A method for obtaining and providing medical information to a device, the method comprising:

receiving, at a main device, medical information from at least one secondary device,
establishing communication to a data processor through a wired or wireless connection;
transmitting medical information to the data processor via a software interface;
displaying the medical information via the data processor;
wherein the medical information comprises at least one of heart rate, respiratory sound information, vibration information and non-respiratory information; and,
wherein the data processor is at least one of a PC, laptop, PDA, Palm, and a mobile phone.

2. A method according to claim 1, wherein the method further comprises comparing the received medical information to at least one of a reference value, a standard value, and a user's unique and personal parameters.

3. A method according to claim 1 or 2, wherein the method further comprises:
calculating a user-specific medical or physiological parameter, standard value or reference value, wherein the value is at least one of BMI, PEF, FEV1, FEV6, FVC and VC.

4. A method according to claim 3, wherein calculating a user specific medical or physiological parameter, standard value, reference value, or user's unique and personal parameters comprises:

providing to an artificial neural network (ANN) an input of the medical information measured;
training the ANN to recognise a given pattern in the user's behaviour,
wherein the given pattern is detected from measurements of normal user breathing and deep user breathing,
wherein detection of the given pattern comprises measuring, calculating and storing at least one of lung sounds, vital capacity and temperature, airflow in, airflow out, FEV1, FEV6, FVC and VC.

5. A method according to claim 4, further comprising:

the ANN producing estimations, calculations, recognition, calibration and correlations of lung sounds, vital capacity and temperature, airflow in, airflow out, FEV1, FEV6, FVC and VC to build and store a user's unique and personal parameters;
providing a prediction of sickness periods to the user by comparing data guessed with data measured practically.

6. A method according to claim 4 or 5, further comprising providing medical information input for the ANN from at least one of: a sound sensor network, a temperature sensor network, biogas sensor network; ultra sound flow sensors, a bidirectional respiratory frequency and flow counter, exhaled breath condensate sensor network, user keyed data and uploaded data.

7. A method according to any of claims 1 to 6, further comprising storing information into a memory or database via a software or hardware interface and/or both locally or an internet server, wherein storing information comprises storing a date and time stamp associated with the information.

8. A method according to any of claims 1 to 7, wherein the secondary device is at least one of an ear device, a sound sensor network, a temperature sensor network, biogas sensor network, ultra sound flow sensors, a bidirectional respiratory frequency and flow counter, exhaled breath condensate sensor network, user keyed data and uploaded data.

9. A method according to any of claims 1 to 8, further comprising detecting asthmatic lungs or lungs with bronchial inflammation, the detecting comprising:

providing the second device with:

a biogas sensor comprising a sensor tip;
an electrode comprising a silver material;
a power supply arranged to provide a positive resting potential of 18 mV to 850 mV;

arranging the sensor tip a small distance from the electrode,
sending medical information from the second device relating to asthmatic lungs or lungs with bronchial inflammation to the main device,
wherein the medical information comprises fractional exhaled nitric oxide and nitric oxide in exhaled breath condensate.

10. A device for obtaining and providing medical information to a user, the device comprising:

a main device;
at least one second device;
a transmitter;
the main device arranged to receive medical information from the at least one secondary device,
the transmitter arranged to transmit for display the medical information to a data processor via a software interface,
wherein the medical information comprises at least one of respiratory sound information and non-respiratory information; and,
wherein the data processor is at least one of a PC, laptop, PDA, Palm, and a mobile phone.

**11.** A device according to claim 10, wherein the at least one second device is at least one of an ear device, a sound sensor network, sound microsensor, a bidirectional air flow detector, a temperature sensor network, biogas sensor network, ultra sound flow sensors, pH sensor, liquid sensor, gas sensor, a bidirectional respiratory frequency and flow counter, exhaled breath condensate sensor network, user keyed data and uploaded data.

**12.** A device according to claim 11, wherein the at least one second device comprises a functionality of at least one of a receiver, a transmitter and a transceiver.

**13.** A device according to any of claims 10 to 12, the device further comprising an artificial neural network (ANN), where in the main device provides an input of the medical information measured by the at least one second device to the ANN,
wherein the ANN is arranged to recognise a given pattern in the user's behaviour, wherein the given pattern is detected from measurements of normal user breathing and deep user breathing,
wherein the detection comprises measuring, calculating and storing at least one of lung sounds, vital capacity and temperature, airflow in, airflow out, FEV1, FEV6, FVC and VC, and
wherein the ANN comprises at least one of back propagation (general solution), fuzzy logic, and simple linear ANN.

**14.** A device according to claim 10 to 13, wherein the device is arranged to detect asthmatic lungs or lungs with bronchial inflammation,
the second device further comprising:

a biogas sensor comprising a sensor tip;
an electrode comprising a silver material;
a power supply arranged to provide a positive resting potential of 18 mV to 850 mV;

the sensor tip being arranged a small distance from the electrode,
the second device arranged to send medical information relating to asthmatic lungs or lungs with bronchial inflammation to the main device,
wherein the medical information comprises fractional exhaled nitric oxide and nitric oxide in exhaled breath condensate.

**15.** A system arranged to provide any of the methods of claims 1 to 9, or comprising the device of claims 10 to 14.

Figure 1A

Figure 1B

Figure 1C

Figure 1D

Figure 2

Figure 3

140
141
143
142

5, 6, 7, 13
Cross Section
Front View

5, 6, 7, 13
Cross Section
Front View
SSN Placed

Figure 4

Figure 5A

Figure 5B

| Date and time | Parameter |
|---|---|
| 07.10.2003 ; 11:05 PM | 64,4567 |
| • • • | • • • |

Figure 6

START  792

CALIBRATION AND
START UP  793

ENTER:
PERSONAL
INFORMATION  795

ENTER:
HEALTH
INFORMATION  797

ENTER:
MEDICAL
INFORMATION  799

DISPLAY:
PERSONAL
INFORMATION  801

DISPLAY:
HEALTH
INFORMATION  803

DISPLAY:
MEDICAL
INFORMATION  805

CORRECT
(CONFIRM)  807  NO

CORRECT
(CONFIRM)  809  NO

CORRECT
(CONFIRM)  811  NO

YES

YES  815

YES

SAVE
PERSONAL
INFORMATION  813

SAVE
HEALTH
INFORMATION

SAVE
MEDICAL
INFORMATION  817

FIGURE 7A

END  819

FIGURE 7B

FIGURE 7C

START 757

Reversibility test with "Bronkodilator" 759

Is increase in $FEV_1$ > 500 ml ?   765

YES

NO

Is increase in $FEV_1$ < 200 ml ?   767

YES

NO

Glukokortikoid Reversibility test   769

Is increase in $FEV_1$ > 200 ml ? (From Glukokortikoid Reversibility test)   775

YES

NO

Set FLAG (Properly Asthma from COPD/Asthma Analysis)   777

Set FLAG (Properly COPD from COPD/Asthma Analysis)   779

STOP   781

FIGURE 7D

34

START

783

Load all of the
measurements

785

Calculate the percentage
deviation of the
measurements from the
standard reference values

787

Set FLAGS that contain the
percentage deviation from
standard for all of
measurements.

789

END

791

# FIGURE 7E

Normal Sound
Child

Bronchial Sound
Child

Tracheal Sound
Adult

FIGURE 7F

FIGURE 8

FIGURE 9A

FIGURE 9B

| CLASS User | CLASS FM_measurement | CLASS PEF_measurement | CLASS FEV_FVC_measurement |
|---|---|---|---|
| **Variables:**<br><br>ID_name<br>Sex<br>Age<br>Height<br>Weight<br>Smoker | **Variables:**<br><br>Temperature<br>Wheezing<br>FMin<br>FMout | **Variables:**<br><br>PEF | **Variables:**<br><br>FEV$_1$<br>FEV$_6$<br>FVC |
| **Functions:**<br><br>Set_data()<br>Get_data()<br>Display_data()<br>Save_data()<br>Calculate_BMI ()<br>Set_refernce_value() | **Functions:**<br><br>Set_data()<br>Get_data()<br>Check_data()<br>Variation_test()<br>Highest_value()<br>Display_data()<br>Save_data() | **Functions:**<br><br>Set_data()<br>Get_data()<br>Check_data()<br>Variation_test()<br>Highest_value()<br>Display_data()<br>Save_data() | **Functions:**<br><br>Set_data()<br>Get_data()<br>Check_data()<br>Variation_test()<br>Highest_value()<br>Display_data()<br>Save_data() |

This is possible to join measurenment classes into one, but preferably
NOT, so it is easy to add/remove/modify the program later

| Main() |
|---|
| **Variables:**<br><br>User<br>FM_measurement<br>PEF_measurement<br>FEV_FVC_measurement |
| **Functions:**<br><br>Display_data() |

**Figure 10**

Input

| date | parameter |
|------|-----------|
| 01.01.03 | 84,567 |
| 02.01.03 | 84,557 |
| . | . |
| . | . |

Pattern recognision

Measurement precision

Personalization (user learning)

Condition precision

FIGURE 11

Classification parameter

Condition prediction

Personalization

Activity planning
Medicine administration
Medicine optimization

Data overview
User recognision
Med. and activity suggestions

FIGURE 12

Sound Sensor Network — 200

Wheezing — 205 — Level (1-9) — 206
Crackles — 207 — Level (1-9) — 208
Grunting — 209 — Level (1-9) — 210
Stridors — 211 — Level (1-9) — 212
Snores — 213 — Level (1-9) — 214

Temperature Sensor Network — 300

Ambient tmp — 305
Body tmp — 306
Device tmp — 307

Ultra Sound Flow Sensors — 320

VC — 325
FM — 326
TC — 327
PEF max — 328

Bidirectional Respiratory Frequency & Flow Counter — 340

FRQ — 345

User Keyed Data — 360

ID — 365
Age — 366
Sex — 367
Height — 368
Weight — 369
Smoking habits — 370

Uploaded Data — 380

Optional Data — 385

A N N — 400

Learn the pattern

Predict the pattern

Correct measurments (error handling)

Predict patient condition

Optimize medicine

450

FIGURE 13

Figure 14A

Figure 14B

Figure 14

**Figure 15**

Fig. 16

1401,
1402
or
1440

1710

1414

Fig. 17

Fig . 18

210 or
211

1

Fig. 19

2010

1414

2010

Fig. 20

Sound-sensor-Transceiver

Sound-sensor-Transceiver

199
Preamplifier

2130
Amp.

2110

2120

Sound-transducer

ADC & DAC
Signal-
processing

Sound-producer

Sound-producer

Sound-transducer

Amp.
2130

Preamplifier
199

Wired / wireless
Connection

555

Interface

2140

ANN

400

**Fig. 21**

**Fig. 22**

1106

1006

Fig. 23

**Fig. 25**

**Fig. 26**

**Fig. 27**

**CLASS User**

Variables:
ID_name
Sex
Age
Height
Weight
Smoker

Functions:
Set_data()
Get_data()
Display_data()
Save_data()
Calculate_BMI ()
Set_refernce
_value()

3202

**CLASS Sound Measuremt**

Variables:
Wheezing
Crackles
Stridors
Grounting
Snores
Pneumonia

Functions:
Set_data()
Get_data()
Check_data()
Variation_test()
Frequency_value()
Display_data()
Save_data()

3204

**CLASS Pressure Measuremt**

Variables:
FVC, FEV₁, FEV₆
PEF

Functions:
Set_data()
Get_data()
Check_data()
Variation_test()
Highest_value()
Display_data()
Save_data()

3206

**CLASS Condensate measuremt**

Variables:
NO, $H_2O_2$, pH

Functions:
Set_data()
Get_data()
Check_data()
Variation_test()
Highest_value()
Display_data()
Save_data()

3208

**CLASS Temperature Measuremt**

Variables:
Device Temp.
Ambient Temp.
User Temp.

Functions:
Set_data()
Get_data()
Check_data()
Variation_test()
Highest_value()
Display_data()
Save_data()

3210

**CLASS Biogas measuremt**

Variables:
FeNO, $O_2$, CO,
$CO_2$

Functions:
Set_data()
Get_data()
Check_data()
Variation_test()
Highest_value()
Display_data()
Save_data()

3212

3220

**Main()**

Variables:
User
Sound
Pressure
Condensate
Temperature
Biogas
Pulse

Functions:
Display_data()

3214

**CLASS Pulse measuremt**

Variables:
Pulse

Functions:
Set_data()
Get_data()
Check_data()
Variation_test()
Highest_value()
Display_data()
Save_data()

**Fig. 28**

Fig. 29

Fig. 30

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **BERGLUND E ; BIRATH G ; BJURE J ; GRIMBY G ; KJELLMAN I ; SANDQUIST I ; SÖDERHOLM B.** Spirometric studies in normal subjects. I. Forced expirograms in subjects between 7-70 years of age. *Acta Med Scand,* 1963, vol. 173, 185-192

- **A J NUNN ; I GREGG.** *New regression equations for predicting peak expiratory flow in adults*